(19) Europäisches Patentamt / European Patent Office / Office européen des brevets

(11) **EP 4 556 055 B1**

(12) **EUROPEAN PATENT SPECIFICATION**

(45) Date of publication and mention
of the grant of the patent:
**08.04.2026 Bulletin 2026/15**

(21) Application number: **24761151.0**

(22) Date of filing: **28.03.2024**

(51) International Patent Classification (IPC):
**G16H 40/40** (2018.01)  **G06N 7/01** (2023.01)
**G01D 21/02** (2006.01)  **G06F 18/20** (2023.01)
**A61N 1/04** (2006.01)  **A61N 1/39** (2006.01)
**A61N 1/08** (2006.01)  **G06N 20/00** (2019.01)
**G06F 123/02** (2023.01)

(52) Cooperative Patent Classification (CPC):
**A61N 1/046; A61N 1/3904; A61N 1/3925;**
**A61N 1/3993; G06F 18/295; G06N 7/01;**
**G06N 20/00; G16H 40/40;** G06F 2123/02

(86) International application number:
**PCT/CN2024/084519**

(87) International publication number:
**WO 2025/066069 (03.04.2025 Gazette 2025/14)**

(54) **DEFIBRILLATION ELECTRODE PAD ASSEMBLY, DEVICE, DEFIBRILLATION ELECTRODE PAD LOSS ESTIMATION METHOD, AND MEDIUM**

DEFIBRILLATIONSELEKTRODENANORDNUNG, DEFIBRILLATIONSVORRICHTUNG UND VERFAHREN ZUR SCHÄTZUNG DES VERSCHLEISSES EINER DEFIBRILLATIONSELEKTRODE UND MEDIUM

ENSEMBLE PATCH D'ÉLECTRODE DE DÉFIBRILLATION, DISPOSITIF, PROCÉDÉ D'ESTIMATION DE PERTE DE PATCH D'ÉLECTRODE DE DÉFIBRILLATION ET SUPPORT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.09.2023 CN 202311263066**
**28.12.2023 CN 202311826856**

(43) Date of publication of application:
**21.05.2025 Bulletin 2025/21**

(73) Proprietors:
• **Primedic (Jiangsu) Medical Science and Technology Co., Ltd.**
**Zhenjiang, Jiangsu 212300 (CN)**
• **Jiangsu Xunjie Medical Science and Technology Co., Ltd.**
**Zhengjiang, Jiangsu 212300 (CN)**
• **Metrax GmbH**
**78628 Rottweil (DE)**

(72) Inventors:
• **JING, Wei**
**Zhenjiang**
**Jiangsu 212300 (CN)**
• **ZHAI, Yinmin**
**Zhenjiang**
**Jiangsu 212300 (CN)**
• **LU, Yu**
**Zhenjiang**
**Jiangsu 212300 (CN)**
• **LV, Haichuan**
**Zhenjiang**
**Jiangsu 212300 (CN)**
• **LIU, Pan**
**Zhenjiang**
**Jiangsu 212300 (CN)**
• **HUANG, Hua**
**Zhenjiang**
**Jiangsu 212300 (CN)**
• **CHEN, Yilin**
**Zhenjiang**
**Jiangsu 212300 (CN)**

• **LIU, Sixing**
  **Zhenjiang**
  **Jiangsu 212300 (CN)**
• **WU, Qun**
  **Zhenjiang**
  **Jiangsu 212300 (CN)**

(74) Representative: **Huang, Liwei**
   **Cäcilienstraße 12**
   **40597 Düsseldorf (DE)**

(56) References cited:
   **CN-A- 103 182 147      CN-A- 104 548 351**
   **CN-A- 109 933 924      CN-A- 110 555 273**
   **CN-A- 114 748 789      CN-A- 114 748 789**
   **CN-A- 117 224 834      CN-A- 117 473 277**
   **US-A- 5 899 925      US-A1- 2002 113 606**
   **US-A1- 2005 277 991      US-A1- 2005 277 991**
   **US-A1- 2017 056 650      US-A1- 2017 312 171**
   **US-A1- 2021 379 392      US-B2- 6 603 318**

**Description**

**CROSS-REFERENCE TO RELATED APPLICATIONS**

The present invention claims priority to the Chinese patent application No. 202311263066.5 filed with CNIPA on September 27, 2023 and entitled "Defibrillation Electrode Assembly and Defibrillation device", and claims priority to Chinese patent application No. 202311826856. X filed with CNIPA on December 28, 2023 and entitled "Defibrillation Electrode Assembly, device, method for estimating defibrillation electrode wear, and Medium".

**TECHNICAL FIELD**

The present invention relates to the field of medical device and technology, particularly relating to a defibrillation electrode assembly, a defibrillation device, and a method for estimating defibrillation electrodes wear and a readable storage medium.

**BACKGROUND**

The present invention in this section only provides background technical information related to the present invention.

85% to 90% of patients with cardiac arrest experience ventricular fibrillation in the early stages. The most effective method for treating ventricular fibrillation is defibrillation by using AED (Automated External Defibrillator) as soon as possible. After the onset of ventricular fibrillation, making defibrillation within 3-5 minutes is most effective. For every 1 minute delay in defibrillation, the survival rate decreases by 7% to 10%. Approximately 540,000 people die from cardiac arrest in China every year. Placing AED emergency device in public places is an important measure to reduce sudden cardiac arrest deaths. AED defibrillation is an important step in cardiopulmonary resuscitation (CPR). The person using AED is the implementer of AED/CPR (hereinafter referred to as the implementer), the person receiving extemal defibrillation with AED is the recipient of AED/CPR (hereinafter referred to as the patient).

At present, AED has been widely used in public places outside hospitals for timely rescue of patients with sudden cardiac arrest. The main structure of AED includes electric energy storage, main control unit, discharging unit, electrode, etc. AED electrode, which is an important part of automatic external defibrillator (AED), is used to transmit electric energy to the patient's heart to restore normal rhythm. AED electrode is generally made of conductive materials and a gel material, with good conductivity and adhesion, and can closely fit on the patient's chest skin. During the research and development of AED electrode, it found that the life (or shelf life) of the electrode was mostly determined by the loss of the gel material. When an AED device was placed in a public place for a long time, affected by the extemal environment and the gas tightness of the AED device itself, the gel material in the AED electrode might fail, and the implementer could not know whether the AED electrode was invalid when he/she needed to use the AED, so the defibrillation effect was reduced when the AED external defibrillation was applied to patients, and the treatment effect for patients was reduced. If the AED electrode was found to have failed after use, it would take one or two minutes to replace the electrode, which is a loss. The survival rate of patients will be reduced.

Given the wide and dispersed distribution of installed defibrillation device, the cost of manually detecting and maintaining defibrillation electrodes is high, and the detection cycle is long. An approach is to monitor the status of defibrillation electrodes by binding their expiration dates on the IOT platform. However, it is not possible to achieve more accurate monitoring by monitoring the performance parameters of the defibrillation electrodes. When the temperature, humidity, and pressure of the environment in which the defibrillation electrodes are located change, or even exceed their set ranges, it can lead to the failure of the defibrillation electrodes. The change of these parameters will also cause the impedance parameters of the conductive gel layer to change, and the gel impedance will be abnormal, and the conductivity will be reduced, making it difficult to detect signals and make defibrillation.

There is a solution that can detect the performance parameters of the defibrillation electrodes. This solution is to overlap two defibrillation electrodes, set the defibrillation layer of the defibrillation electrodes oppositely, and input the alternating current to detect the gel impedance. This detection method requires the two defibrillation electrodes to be fixed and installed in a superposition manner. Before use, the defibrillation electrodes must be separated which would reduce the rescue speed. Moreover, due to the large volume of the humidity sensor itself, placing it on the defibrillation electrode for emergency treatment of the human body will increase the weight and size of the electrode, affecting the defibrillation effect of the defibrillation electrode.

Therefore, at present, there is a lack of technical means for continuous measurement and display of the life defibrillation electrode wear. How to solve this problem and how to effectively monitor the status of defibrillation electrode while maintaining its working performance, have become the topics that the present invention aims to study and solve.

US 2005/277991 A1 discloses an apparatus and techniques for determining whether a medical electrode, such as a defibrillation electrode coupled to an automated external defibrillator, is in a condition for replacement. The determination can be made as a function of one or more data. In one exemplary embodiment, the determination is a function of one or more measurements of an impedance of a hydrogel bridge in a test module. In another exemplary embodiment, the determination is a function of one or more environmental condition data from one or more environmental sensors.

## SUMMARY

The purpose of the present invention is to provide a defibrillation electrode assembly, a defibrillation device, a method for estimating defibrillation electrode wear and a medium.

To achieve the above objectives, the first aspect of the present invention proposes a method for estimating defibrillation electrode wear. This method includes:

S100, setting an environmental container for estimating electrochemical attenuation of a gel material, and for estimating the defibrillation electrode wear, determining environmental variables and activation energy failure mechanism variables as the hidden parameters related to gas tightness of electrochemical attenuation estimation of the gel material, wherein the environmental container is equipped with humidity sensors and gas pressure sensors for observing humidity and gas pressure sequences.

S200, establishing a hidden Markov model and using it to establish a hidden Markov process for hidden parameters related to gas tightness under observed humidity and gas pressure sequences, wherein the observed values of humidity and gas pressure sequences are used as the observation vectors of the hidden Markov chain and are defined as the collection of humidity and gas pressure observation sequences, and hidden parameters related to gas tightness are used as the hidden state vectors of the hidden Markov chain and are defined as a gas-tightness hidden state sequence;

S300, learning for the hidden Markov model and adjusting parameters of the model using collected multiple sets of humidity and gas pressure observation sequences, wherein the set of optimal model parameter obtained from each set of humidity and gas pressure observation sequences is determined as determined model parameters;

S400, decoding the hidden Markov model and estimating the defibrillation electrode wear, wherein the decoding is based on the determined model parameters and the humidity and gas pressure observation sequence corresponding to the humidity and gas pressure sequence of the previous moment when using the device to obtain the hidden parameters related to gas tightness corresponding to the current gas-tightness hidden state sequence and wherein the environmental variables and activation energy failure mechanism variables corresponding to the hidden parameters related to gas tightness are used to estimate the life measurement of the gel material, and the estimated current defibrillation electrode wear value is finally obtained and displayed.

The second aspect of the present invention proposes a defibrillation electrode assembly for intelligent display of defibrillation electrode wear. The defibrillation electrode assembly includes a defibrillation electrode and an island electrode arranged in a sealed environmental container. The environmental container is equipped with a humidity sensor, a gas pressure sensor for observing humidity and gas pressure sequences, and a temperature sensor for observing temperature values. The humidity sensor, pressure sensor, and temperature sensor are connected to a detection module, and the detection module estimates the defibrillation electrode wear based on the method for estimating defibrillation electrode wear described in the first aspect of the present invention using parameters obtained from the humidity sensor, pressure sensor, and temperature sensor. The estimation results are displayed through the display module.

A third aspect of the present invention proposes a defibrillation device. The defibrillation device includes a defibrillation electrode assembly as described in the second aspect of the present invention, and a defibrillator electrically connected to the assembly. The defibrillation device can intelligently display defibrillation electrode wear.

The fourth aspect of the present invention proposes a readable storage medium on which a control program is stored. When the control program is executed by a detection module, the detection module executes the steps of the method for estimating defibrillation electrode wear as described in the first aspect.

The fifth aspect of the present invention proposes another defibrillation electrode assembly. An island electrode is placed in a sealed packaging bag where the defibrillation electrode is placed. By detecting the island electrode to provide feedback on the performance parameters of the defibrillation electrode, the monitoring accuracy is high and the defibrillation effect is improved.

The technical solution adopted in the fifth aspect of the present invention is to design a defibrillation electrode assembly to include: two defibrillation electrodes located in a sealed packaging bag, and an island electrode used to feedback on the performance parameters of the defibrillation electrodes. The two defibrillation electrodes are connected to an external connection end through a defibrillation discharging high-voltage line, and the island electrode is placed in a sealed packaging bag. The island electrode is equipped with a detection module, which is connected to the external connection end.

The sixth aspect of the present invention also proposes a defibrillation device, which has the defibrillation electrode assembly provided in the fifth aspect mentioned above.

The relevant content of the present invention is explained as follows.

1. Through the implementation of the above technical solution of the present invention, in view of the characteristics that the life defibrillation electrode wear is directly related to the gel material and is affected by the extemal environment and the gas tightness of the AED device itself, an environmental container is set for the electrochemical attenuation estimation of the gel material, and the environmental container is equipped with a humidity sensor and a gas pressure sensor for observing the humidity and gas pressure sequence. That is, the continuous and regular humidity and gas pressure observation are conducted during the operation of the device and are collected and listed as the humidity and gas pressure sequence. Further, for estimating the defibrillation electrode wear, activation energy failure mechanism variables and environmental variables related to the life of the gel material are selected as the hidden parameters related to gas tightness for the electrochemical attenuation estimation of the gel material. In this step, the sealing requirements for the daily storage of defibrillation electrodes are considered. The influence of the sealing environment on the activation energy failure mechanism variables and the environmental variables related to the life of a gel material is also considered. The rightness and reference value of the estimated electrode wear is ensured. Since the life state of the gel material of the defibrillation electrode only depends on the current state and extemal environmental factors, and has nothing to do with the previous state, and the environmental variables and activation energy failure mechanism variables cannot be directly observed, a hidden Markov model is established to express the hidden state of the hidden parameters related to gas tightness affected by the gas tightness in the electrochemical accelerated attenuation estimation of the gel by using the measured humidity and gas pressure sequences. A correlation model related to the humidity and gas pressure observation sequences, the gas-tightness hidden state series, and the hidden parameters related to gas tightness is established so that the process of electrode wear estimation has a model basis. Then leaming for the hidden Markov model to optimize the model, so as to provide the performance of the model and improve the accuracy of the model output. Finally, decoding is done according to the humidity and gas pressure observation sequence corresponding to the humidity and gas pressure sequence at the previous time when using the device and the detennined model parameters, so as to obtain the current hidden parameters related to gas tightness of the corresponding gas-tightness hidden state sequence related to the life state of the gel material of the defibrillation electrode, and then this parameter is used to estimate the life measurement of the gel material, and finally the estimated current defibrillation electrode wear value is obtained and displayed, so that the rescuer can directly know the defibrillation electrode wear value from the display module when using the AED device (or can converted into the current validity period and service life of the defibrillation electrode). This is used to detennine whether the defibrillation electrode needs to be replaced, in order to ensure that the defibrillation electrode has good conductivity and adhesion during AED extemal defibrillation for patients, ensure that the current can be accurately transmitted to the heart for defibrillation. It enables rescuers to correctly use AED device for AED extemal defibrillation without delay, racing against time to improve patient survival rate. This can quickly and effectively provide CPR first aid to patients, improve patient survival rate and emergency effectiveness, and further ensure the safety and health of patients.

2. In the first aspect of the above technical solution, in step S100, the environmental container is also equipped with a temperature sensor for observing temperature values. In step S400, the value of defibrillation electrode wear is estimated based on the temperature value. The reliability is increased by additionally use the temperature value to estimate the defibrillation electrode wear.

3. In the first aspect of the above technical solution, the following reaction formula is used to estimate the life loss of the electrode mainly based on the loss of activation energy of gel material:

$$F = Ce^{-\frac{E}{kB*M}}$$

In the formula, F is the chemical reaction rate of gel material, and the life of the defibrillation electrode can be calculated as the reciprocal of the chemical reaction rate of gel material, that is, 1/F; C represents the environmental variable; e represents the natural logarithmic base; E represents the activation energy failure mechanism variable; kB is the Boltzmann constant; M represents temperature.

4. In the first aspect of the above technical solution, in step S200, the following steps are included:

S210, defining each tuple in the hidden Markov model, wherein each tuple includes a set of humidity and gas pressure observation sequences, a hidden state set, a state probability matrix, an emission probability matrix, and an initial probability matrix;

S220, establishing a hidden Markov model, which is related to the state probability matrix, emission probability matrix, and initial probability matrix;

S230, performing hidden Markov process representation, which represents the output probability of the humidity and gas pressure observation sequence under the hidden Markov model, i.e. the joint probability of the humidity and gas pressure observation sequence and the gas-tightness hidden state representation occurring simultaneously under the hidden Markov model.

Through the implementation of the above established hidden Markov model, it can effectively correlate the hidden parameters related to gas tightness of the corresponding gas-tightness hidden state sequence related to the life state of the gel material of the defibrillation electrode with the humidity gas pressure observation sequence from timely measured humidity time sequences and gas pressure time sequences. A joint probability of the simultaneous occurrence of the humidity pressure observation sequence and the gas-tightness hidden state expression under the hidden Markov model is expressed by the hidden Markov process, and this can provide a basis for the subsequent optimization of the model and the output of the model, to ensure the accuracy of the model output, and to ensure that the obtained hidden parameters related to gas tightness are consistent and correct with the relevant variables of the gel material under the current state.

5. In the first aspect of the above technical solution, in step S200, the following steps are included:

S210, defining the tuples in the hidden Markov model:

S211, defining a set of humidity and gas pressure observation sequences, using the humidity and gas pressure observation sequences $\mathbf{O} = [o_1, o_2,..., o_T]$ of the timely measured humidity time sequence and gas pressure time sequence as the observation vectors of the hidden Markov chain, and defining a set of humidity and gas pressure observation sequences, wherein T represents the period during which the humidity sensor and gas pressure sensor start to measure at a fixed time when the equipment is working, : $o_t = [pressure_t, humidity_t]^T$ is observation values of the gas pressure and humidity measured at time t.

S212, defining a set of hidden states, representing the gas-tightness hidden states as gas-tightness hidden state sequence Q of a Markov chain, and defining the gas-tightness hidden state sequence as $\mathbf{Q} = [q_1, q_2,..., q_T]$, wherein $q_t = [C_t, E_t]^T$ is the hidden representation of the hidden parameters related to gas tightness at the time t;

S213, defining a state probability matrix: for the state transition set of gas-tightness hidden states at different times, it is represented as the state probability matrix A, wherein each element $a_{ij}$ of the state probability matrix A represents the transition probability from state i to state j;

S214, defining an emission probability matrix: for the set of possible humidity and gas pressure measurement values generated in the gas-tightness hidden state, it is represented as the emission probability matrix B. The elements $b_{qi}$ of the emission probability matrix B represent the emission probability from the hidden state $q_i$ to the observed state;

S215, defining the initial probability matrix: for the expression of the factory default of initial gas-tightness hidden state, it is represented as the initial probability matrix $\Pi$, wherein each element $\pi_{qi}$ in the matrix represents the initial probability of the hidden state $q_i$, that is, the factory-default probability distribution;

S220, establishing a hidden Markov model, which is $\lambda = \{\mathbf{A}, \mathbf{B}, \Pi\}$;

S230, performing hidden Markov process representation which is the output probability of the humidity and gas pressure observation sequence $\mathbf{O}$ in the model $\lambda = \{\mathbf{A}, \mathbf{B}, \Pi\}$, wherein $P(O|\lambda)$ represents the joint probability of the humidity and gas pressure observation sequence $\mathbf{O}$ and the gas-tightness hidden state sequence $\mathbf{Q} = [q_1, q_2,..., q_T]$ occurring simultaneously in the model $\lambda$. The formula is as follows:

$$P(O|\lambda) = \sum_Q P(O|Q, \lambda) = \sum_{q_1, q_2,...q_T} \pi_{q_1} b_{q_1}(o_1) a_{q_1 q_2} b_{q_2}(o_2)...a_{q_{T-1} q_T} b_{q_T}(O_T)$$

For a fixed hidden state $\mathbf{Q} = [q_1, q_2, ... , q_T]$, the probability of obtaining a humidity and gas pressure observation sequence $\mathbf{O} = [o_1, o_2,..., o_T]$ is $P(O|Q, \lambda) = b_{q_1}(o_1)b_{q_2}(o_2)...b_{q_T}(o_T)$.

Through the implementation of the hidden Markov model established above, the accuracy, efficiency, and precision of the model have been further enhanced. A more reasonable and accurate model building process has been adopted to provide assurance for subsequent wear estimation.

6. In the first aspect of the above technical solution, in step S300 learning for the hidden Markov model, the following steps are adopted:

S310, collecting k groups of humidity and gas pressure observation sequences $\mathbf{O} = [\mathbf{O}^1, \mathbf{O}^2,..., \mathbf{O}^K]$ , wherein

$\mathbf{O^k} = [O_1^k, O_2^k, \ldots, O_T^k]$ represents the $k^{th}$ group humidity and gas pressure observation sequence;

S320, obtaining the model parameters which makes maximal joint probability value with the $k^{th}$ group humidity and gas pressure observation sequence $O^k$ and define the model parameters as optimal;

8330, training by using K groups of humidity and gas pressure observation sequences to obtain the optimal parameter set with the given humidity and gas pressure observation sequence O;

S340, adjusting to make the joint probability of simultaneous occurrence of humidity and gas pressure observation sequences **O** and gas-tightness hidden state sequences $\mathbf{Q} = [q_1, q_2, \ldots, q_T]$ under the model $\lambda$ maximized, and setting the optimal model parameters obtained with each humidity and gas pressure observation sequence as determined model parameters.

By implementing the learning process for the hidden Markov model mentioned above, the accuracy of the hidden parameters related to gas tightness obtained during subsequent model decoding can be effectively improved, thereby enhancing the performance of the model.

7. In the first aspect of the above technical solution, in step S400, the following steps are adopted to obtain the parameters represented by the gas-tightness hidden state of the corresponding hidden parameters related to gas tightness:

S410, among the detennined model parameters, defining $\delta_t(i)$ as the maximum probability for generating $o_1, o_2, \ldots, ot$ along a path $q_1, q_2, \ldots, q_T$ at time t;

S420, initializing $\delta_t(i) = \pi_i b_i(o_i)$;

S430, calculating the results $\delta_t(j) = \max_j (\delta_{t-1}(i)a_{ij}|b_j(o_t))$ of the humidity and gas pressure observation sequence corresponding to the previous humidity and gas pressure sequence on the next path j;

S440, taking the maximum value in the S430 path as the output result $P(\mathbf{Q}|\lambda) = \max_j (\delta_{t-1}(i)a_{ij})$, wherein $P(\mathbf{Q}|\lambda)$ is the parameter represented by the environmental variable C and the activation energy failure mechanism variable E under the influence of gas tightness.

Through the implementation of the above specific process of obtaining the parameters indicated by the gas-tightness hidden state of the corresponding hidden parameters related to gas tightness, the humidity and gas pressure observation sequence corresponding to the humidity and gas pressure sequence collected at the previous time when using the equipment can be effectively and correctly used according to the fact that the life state of the gel material of the defibrillation electrode only depends on the current state and the external environmental factors. The output result taken is the maximum value in the path of step S430, so that the obtained hidden parameters related to gas tightness are the closest and most consistent with the current state of the gel material, and are the most representative. The electrode wear value obtained by estimation will also be the closest to real, so as to provide the rescuer with the most accurate and real value of defibrillation electrode wears, ensuring the patient's AED defibrillation in vitro.

8. In the second aspect of the above technical solution, the detection module comprises a controller, the humidity sensor is connected to the controller through a data processing module, the temperature sensor is connected to the controller through a series resistor voltage divider module, and the pressure sensor is connected to the controller. In this way, the detection module can obtain correct, accurate, and effective relevant detection values, providing hardware support for the implementation of the defibrillation electrode wear estimation method.

9. In the second aspect of the above technical solution, the island electrode comprises two test sheets made of the same material as the defibrillation electrode. The defibrillation layers of the two test sheets are stacked in place, and the impedance test points of the two test sheets are connected to the detection module to form an impedance detection circuit for inputting direct current. Except size, the structure and material of the test sheet are the same as those of the defibrillation electrode. Therefore, the monitored gel impedance parameters of the island electrode can be completely equivalent to the gel impedance test value of the defibrillation electrode after conversion of size differences.

10. In the second aspect of the above technical solution, the detection module is equipped with a controller, the humidity sensor is connected to the IIC communication channel of the controller through a data processing module, the temperature sensor is connected to the second ADC channel of the controller through a voltage divider module of series resistance, the impedance test point is connected in series with an impedance test bridge and an instrument amplifier to the third ADC channel of the controller, the gas pressure sensor is connected to the serial communication channel of the controller, and the display screen is connected to the SPI communication port of the controller; wherein the controller converts the analog signals of each ADC channel into digital signals.

11. In the second aspect of the above technical solution, the temperature sensor adopts a thermistor, which is built-in in the environmental container or placed on the detection module.

12. In the second aspect of the above technical solution, the island electrode is installed inside the environmental container and connected to the detection module through a detection connection line.

13. In the second aspect of the above technical solution, the humidity sensor and/or gas pressure sensor are installed inside the environmental container and connected to the detection module through a detection connection wire, or directly installed on the detection module.

14. In the second aspect of the above technical solution, the area of the island electrode is smaller than that of the defibrillation electrode.

15. In the third aspect of the above technical solution, the display module is located on at least one selected from the detection module, the defibrillator, a rescuer mobile terminal, or an emergency platform.

16. In the third aspect of the above technical solution, the detection module is connected to the electrode plug through a communication line and then inserted into the defibrillator. The defibrillator is located outside the environmental container, and the communication line extends from the detection module and is connected to the defibrillator through the electrode plug.

Due to the above solution, the present invention has the following advantages and effects compared to the existing technology:

1. Through the implementation of the technical solution of the present invention, an environmental container for electrochemical attenuation estimation of a gel material is set, and the environmental container is equipped with a humidity sensor and a gas pressure sensor for observing the humidity and gas pressure sequence, that is, during the operation of the device, continuous and timed humidity and gas pressure observations are conducted, and the humidity and gas pressure sequences are collected and listed as humidity and gas pressure sequences. Then, for estimating the defibrillation electrode wear, activation energy failure mechanism variables and environmental variables related to the life of the gel material are selected as the hidden parameters related to gas tightness for electrochemical attenuation estimation of the gel material. In this step, both the sealing requirements for daily storage of defibrillation electrodes and the effect of sealed environment on the environmental variables and activation energy failure mechanism variables related to the life of the gel material are considered, to ensure the accuracy and reference value of the estimated electrode weares.

2. Through the implementation of the technical solution of the present invention, since the life state of the gel material of the defibrillation electrode only depends on the current state and external environmental factors, and has nothing to do with the previous state, and the environmental variables and activation energy failure mechanism variables cannot be directly observed, hidden Markov model is established to express the hidden state of the hidden parameters related to gas tightness affected by the gas tightness in the electrochemical accelerated attenuation estimation of the gel through the measured humidity and gas pressure sequences, and to establish a correlation model of the humidity and gas pressure observation sequence, the gas-tightness hidden state sequence, and the hidden parameters related to gas tightness, so as to provide a model basis for the process of electrode wear estimation. A correct and reliable model is designed to help the estimation of electrode wear. Learning for the hidden Markov model to optimize the model provides the performance of the model and improves the accuracy of the model output.

3. Through the implementation of the technical solution of the present invention, decoding is done according to the humidity and gas pressure observation sequence corresponding to the humidity and gas pressure sequence at the previous moment when using the device and detennine model parameters, so as to obtain the hidden parameters related to gas tightness of the current corresponding gas-tightness hidden state sequence related to the life state of the gel material of the defibrillation electrode, and then estimate the life measurement of the gel material with this parameter, finally obtain the estimated current defibrillation electrode wear value and display, so that when using the AED equipment, the rescuer can directly know the defibrillation electrode wear value from the display module (or can be converted into the current validity period and service life of the defibrillation electrode), so as to determine whether it is necessary to replace the defibrillation electrode.

4. To sum up, the implementation of the above solution has built a stable environment, used accurate and highly relevant parameters as the basis for model building and optimized learning, and can obtain the current hidden parameters related to gas tightness corresponding to the gas-tightness hidden state sequence based on the key point that the life state of the gel material of the defibrillation electrode only depends on the current state and extemal environmental factors. Thus, the estimated current defibrillation electrode wear value is closest to the current state of the gel material. The technical blank of continuous measurement and display of the life defibrillation electrode wear is filled. This can ensure that the defibrillation electrode has good electrical conductivity and adhesion and ensures that the current can be accurately transmitted to the heart for defibrillation. This can allow rescuers to use AED equipment correctly for AED defibrillation without delay, racing against time, in order to improve patient survival rate, quickly and effectively provide CPR emergency treatment, improve patient survival rate and effectiveness, and further ensure patient safety and health protection.

5. Compared with existing technology, the present invention provides an island electrode in a sealed packaging bag where the defibrillation electrode is located. By monitoring the various parameters of the island electrode through a detection module, the performance parameters of the defibrillation electrode can be calculated or directly read. The monitoring accuracy is high, and the island electrode, together with the detection module, is completely independent of and outside the defibrillation electrode. The wiring of the defibrillation electrode is fewer, the defibrillation electrode is lighter and thinner, and it is less affected during operation, improving the defibrillation effect and has higher practicality.

## BRIEF DESCRIPTION OF THE DRAWINGS

The following provides a detailed explanation of the present invention in conjunction with embodiments and drawings, wherein:

Fig.1 is a schematic flow chart of the method for estimating defibrillation electrode wear according to a first embodiment of the present invention;
Fig.2 is a schematic diagram of establishing a hidden Markov process according to the first embodiment of the present invention;
Fig.3 is a schematic diagram of the distribution of the defibrillation electrode assembly according to a second embodiment of the present invention;
Fig.4 is a schematic diagram of the wiring connection of the defibrillation device according to a third embodiment of the present invention;
Fig.5 is a schematic diagram of the distribution of the defibrillation electrode assembly in the packaging bag according to a fifth embodiment of the present invention;
Fig.6 is a schematic diagram of the circuit connection of the defibrillation electrode assembly according to the fifth embodiment of the present invention.

Reference number: 1. defibrillation electrode; 11. left defibrillation electrode; 12. right defibrillation electrode; 13. defibrillation discharging high-voltage line; 2. island electrode; 21. impedance detection line; 22. temperature detection line; 23. humidity detection line; 24. gas pressure detection line; 3. external connection end; 4. temperature sensor; 5. humidity sensor.

## DETAILED DESCRIPTION OF THE EMBODIMENTS

In order to make the above objectives, features, and advantages of the present invention more obvious and understandable, a detailed explanation of the specific embodiment of the present invention will be provided below in conjunction with the accompanying drawings. Many specific details are elaborated in the following description to facilitate a thorough understanding of the present invention. Therefore, the present invention is not limited by the specific embodiments disclosed below.

The language used in this article is only to describe specific embodiments and is not intended as a limitation of the present invention. Singular forms such as "one" or "this", as used in this article, also include plural forms.

The terms "first", "second", etc. used in this article do not specifically refer to order, sequence or ranking, nor are they intended to limit the present invention. They are only used to distinguish components or operations described by the same technical tenns.

The term "connection" or "positioning" used in this article can refer to two or more components or devices directly making physical contact with each other, or indirectly making physical contact with each other, and can also refer to two or more components or devices operating or acting on each other.

The terms "including", "comprising", "having", etc. used in this article are all open-ended terms, meaning to include but not limited to.

Regarding the terms used in this article, unless otherwise specified, they usually have the ordinary meaning of each term used in the field, in the content of this case, and in special content. Certain terms used to describe the present invention will be discussed below or elsewhere in this description to provide additional guidance for those skilled in the art.

The purpose of the embodiment of the present invention is to provide a technical solution that can timely detect and estimate the defibrillation electrode wear, and that the rescuer can directly know the electrode wear when using defibrillation equipment, in order to fill the technical gap in continuous measurement and display of the lifespan defibrillation electrode wear. The rescuer can determine whether to replace the defibrillation electrode after knowing the value of the defibrillation electrode wear, so as to ensure that the defibrillation electrode has good conductivity and adhesion when performing AED external defibrillation for patients, ensure that the current can be accurately transmitted to the heart for defibrillation, and enable the rescuer to correctly use the AED equipment for AED external defibrillation without delay, racing against time, in order to improve patient survival rate. CPR emergency treatment can be provided to patients quickly and effectively, patient survival rate and emergency response effect can be improved, and the safety and health of patients can be further ensured.

**First embodiment**

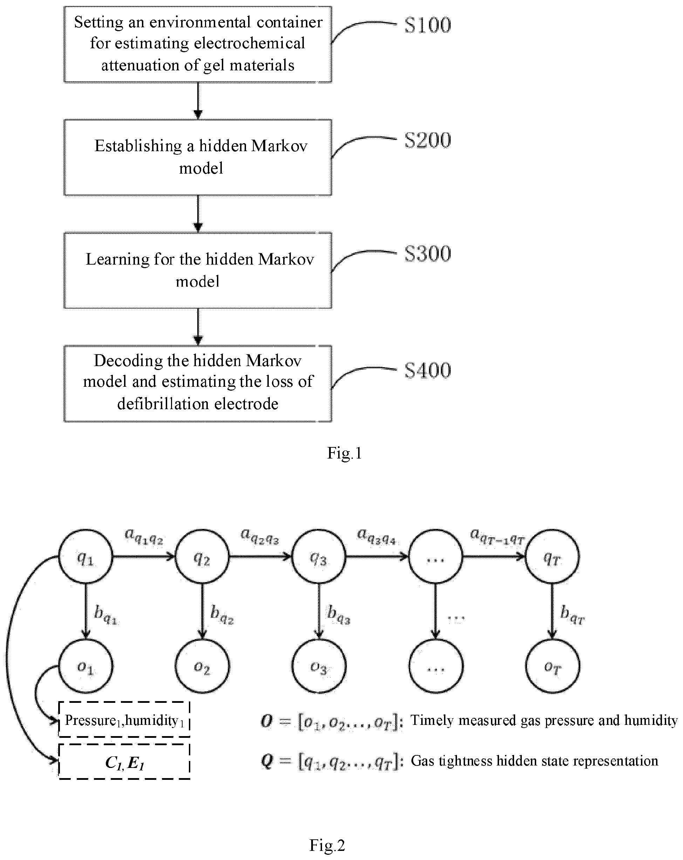

In view of the characteristics that the life defibrillation electrode wear is directly related to the gel material and is affected by the external environment and the gas tightness of the AED equipment itself, the first embodiment of the present invention proposes a method for estimating defibrillation electrode wear. As shown in Fig.1, the method for estimating the defibrillation electrode wear includes the following steps:

S100, setting an environmental container for estimating electrochemical attenuation of a gel material, and determining, for estimating the defibrillation electrode wear, environmental variables and activation energy failure mechanism variables as the hidden parameters related to gas tightness of electrochemical attenuation estimation of the gel material, wherein the environmental container is equipped with humidity sensors and gas pressure sensors for observing humidity and gas pressure sequences.

S200, establishing a hidden Markov model and using it to establish a hidden Markov process for hidden parameters related to gas tightness under observed humidity and gas pressure sequences, wherein the observed values of humidity and gas pressure sequences are used as observation vectors of hidden Markov chain and are defined as a set of humidity and gas pressure observation sequences, and hidden parameters related to gas tightness are used as the hidden state vectors of the hidden Markov chain and are defined as the gas-tightness hidden state sequences;

S300, learning for the hidden Markov model and adjusting parameters of the model using collected multiple sets of humidity and gas pressure observation sequences, wherein a set of optimal model parameters obtained from each set of humidity and gas pressure observation sequences is determined as detennined model parameters;

S400, decoding the hidden Markov model and estimating the defibrillation electrode wear, wherein the decoding is based on the determined model parameters and the humidity and gas pressure observation sequence corresponding to the humidity and gas pressure sequence of the previous moment when using a device to obtain the hidden parameters related to gas tightness corresponding to the current gas-tightness hidden state sequence and wherein the environmental variables and activation energy failure mechanism variables corresponding to the hidden parameters related to gas tightness are used to estimate the life measurement of the gel material, and the estimated current defibrillation electrode wear value is finally obtained and displayed.

In the step S100 of setting an environmental container for electrochemical attenuation estimation of a gel material, in view of the characteristics that the life defibrillation electrode wear is directly related to the gel material and is affected by the external environment and the gas tightness of the AED device itself, the environmental container is equipped with a humidity sensor and a gas pressure sensor for observing the humidity and gas pressure sequence. That is, the continuous and regular humidity and gas pressure observation are conducted during the operation of the device and are collected and listed as the humidity and gas pressure sequence. Further, for estimating the defibrillation electrode wear, activation energy failure mechanism variables and environmental variables related to the life of the gel material are selected as the hidden parameters related to gas tightness for the electrochemical attenuation estimation of the gel material. In this step, the sealing requirements for the daily storage of defibrillation electrodes are considered. The influence of the sealing environment on the activation energy failure mechanism variables and the environmental variables related to the life of the gel material is also considered. The rightness and reference value of the estimated electrode wear is ensured.

In the step S200 of establishing a hidden Markov model as mentioned above, taking into consideration the fact that the life state of the gel material of the defibrillation electrode only depends on the current state and external environmental factors, has nothing to do with the previous state, and the environmental variables and activation energy failure mechanism variables cannot be directly observed, a hidden Markov model is established to express the hidden state of the hidden parameters related to gas tightness affected by the gas tightness in the electrochemical accelerated attenuation estimation of the gel using the measured humidity and gas pressure sequences. A correlation model related to the humidity and gas pressure observation sequences, the gas-tightness hidden state series, and the hidden parameters related to gas tightness is established so that the process of electrode wear estimation has a model basis. A correct and reliable model is designed to assist in estimating electrode weares.

In the Step S300 of leaming for the hidden Markov model, by the leaming for the hidden Markov model, the model is optimized, so as to provide the performance of the model and improve the accuracy of the model output.

In the step S400 as mentioned above of decoding the hidden Markov model and estimating the defibrillation electrode wear , decoding is done according to the humidity and gas pressure observation sequence corresponding to the humidity and gas pressure sequence at the previous time when using the device and the detennined model parameters, so as to obtain the current hidden parameters related to gas tightness of the corresponding gas-tightness hidden state sequence related to the life state of the gel material of the defibrillation electrode, and then this parameter is used to estimate the life measurement of the gel material, and finally the estimated current defibrillation electrode wear value is obtained and displayed, so that the rescuer can directly know the defibrillation electrode wear value from the display module when using the AED device (or can converted into the current validity period and service life of the defibrillation electrode). This is used to detennine whether the defibrillation electrode needs to be replaced.

In the first embodiment of the present invention, in step S100, the environmental container is also equipped with a temperature sensor for observing temperature values. In step S400, the defibrillation electrode wear value is estimated additionally based on the temperature value. The reliability is increased by additionally using the temperature value to estimate the defibrillation electrode wear.

In the first embodiment of the present invention, in step S200, the following steps are included:

S210, defining each tuple in the hidden Markov model, wherein each tuple includes a set of humidity and gas pressure observation sequences, a hidden state set, a state probability matrix, an emission probability matrix, and an initial probability matrix;

S220, establishing a hidden Markov model, which is related to the state probability matrix, emission probability matrix, and initial probability matrix;

S230, performing hidden Markov process representation, which represents the output probability of the humidity and gas pressure observation sequence under the hidden Markov model, i.e. the joint probability of the humidity and gas pressure observation sequence and the gas-tightness hidden state representation occurring simultaneously under the hidden Markov model.

Through the implementation of the establishing hidden Markov model above, it can effectively correlate the hidden parameters related to gas tightness of the corresponding gas-tightness hidden state sequence related to the life state of the gel material of the defibrillation electrode with the humidity gas pressure observation sequence from timely measured humidity time sequences and gas pressure time sequences. A joint probability of the simultaneous occurrence of the humidity pressure observation sequence and the gas-tightness hidden state expression under the hidden Markov model is expressed by hidden Markov process. This can provide a basis for the subsequent optimization of the model and the output of the model, to ensure the accuracy of the model output, and to ensure that the obtained hidden parameters related to gas tightness are consistent with the relevant variables of the gel material under the current state and are right.

In the first embodiment of the present invention, the step S300 of learning for the hidden Markov model comprises the following steps:

S310, collecting K groups of humidity and gas pressure observation sequences $\mathbf{O} = [\mathbf{O}^1, \mathbf{O}^2,..., \mathbf{O}^K]$, wherein $\mathbf{O^k} = [o_1^k, o_2^k, \ldots, o_T^k]$ represents the $k^{th}$ group humidity and gas pressure observation sequence;

S320, obtaining the model parameters which makes maximal joint probability value with the $k^{th}$ group humidity and gas pressure observation sequence $O^k$ and define the model parameters as optimal parameter sets;

S330, training by using K groups of humidity and gas pressure observation sequences to obtain the optimal parameter set with the given humidity and gas pressure observation sequence O:

S340, adjusting to make the joint probability of simultaneous occurrence of humidity and gas pressure observation sequences $\mathbf{O}$ and gas-tightness hidden state sequences $\mathbf{Q} = [q_1, q_2,...,q_T]$ under the model $\lambda$ maximum, and setting the optimal model parameters obtained with each humidity and gas pressure observation sequence as determined model parameters.

By implementing the learning process for the hidden Markov model as mentioned above, the accuracy of the hidden parameters related to gas tightness obtained during subsequent model decoding can be effectively improved, thereby enhancing the performance of the model.

In the first embodiment of the present invention, in step S400 for obtaining the parameters represented by the gas-tightness hidden state of the corresponding hidden parameters related to gas tightness, the following steps are adopted:

S410, among the determined model parameters, defining $\delta_t(i)$ as the maximal probability for generating $o_1, o_2,..., o_t$ along a path $q_1, q_2,..., q_T$ at time t;

S420, initializing $\delta_t(i) = \pi_i b_i(o_i)$;

S430, calculating the results $\delta_t(j) = \max_j (\delta_{t-1}(i)a_{ij}|b_j(o_t))$ of the humidity and gas pressure observation sequence corresponding to the previous humidity and gas pressure sequence on the next path j;

S440, taking the maximum value in the step S430 path as the output result $P(\mathbf{Q}|\lambda) = \max_j \left(\delta_{t-1}(i) a_{ij}\right)$,

wherein $P(Q|\lambda)$ is the parameter represented by the environmental variable C and the activation energy failure mechanism variable E under the influence of gas tightness.

Through the implementation of the above specific process of obtaining the parameters indicated by the gas-tightness hidden state of the corresponding hidden parameters related to gas tightness, the humidity and gas pressure observation sequence corresponding to the humidity and gas pressure sequence collected at the previous time when using the equipment can be effectively and correctly used according to the fact that the life state of the gel material of the defibrillation electrode only depends on the current state and the external environmental factors. The output result taken is the maximum value in the path of step S430, so that the obtained hidden parameters related to gas tightness are the closest and most consistent with the current state of the gel material, and are the most representative. The electrode wear value obtained by estimation based on this will also be the closest to real, so as to provide the rescuer with the most accurate and real value of defibrillation electrode wears, ensuring the patient's AED defibrillation in vitro.

A more specific and detailed example as below will be used to further explain the first embodiment of the present invention.

In this detailed example, during the operation of the device, the life defibrillation electrode wear mainly made of a gel material is estimated using the humidity, gas pressure and temperature measured continuously and regularly. The method is to establish a hidden Markov model to represent the hidden state of the hyperparameters affected by gas tightness in the electrochemical acceleration attenuation formula by observed humidity and gas pressure sequence, and estimate the electrode life using the electrochemical acceleration attenuation formula in combination with temperature.

For the implementation of this detailed example, the present invention designs and uses humidity sensors and gas pressure sensors to detect the sealing environment of environmental containers. Further, a hidden Markov model is used to establish a hidden Markov process for hidden parameters related to gas tightness with observed humidity and gas pressure sequences. The hidden state representation of hyperparameters (environmental variables, activation energy failure mechanism variables) affected by gas tightness in the Arrhenius formula is calculated to achieve estimation of electrode life loss.

Before the defibrillation device leaves the factory, use the Baum Welch algorithm to learn for the hidden Markov model and obtain a set of hidden Markov model parameters.

During the use of defibrillation device, the Viterbi algorithm is used to decode the hidden Markov model and the hidden representation of the hidden parameters related to gas tightness (environmental variables, activation energy failure mechanism variables) with the humidity and gas pressure sequence measured by the sensor that is started at a fixed time can be obtained. The life measurement of gel material components is estimated using Arrhenius formula.

In the process of setting an environmental container for electrochemical attenuation estimation of a gel material in step S100 of the detailed example, the activation energy of gel is directly affected by the internal temperature of the defibrillation electrode. The temperature change will lead to the reduction of the bonding viscosity between the electrode and the human body, resulting in the loss of component life. Arrhenius formula is used to estimate the life loss of the electrode dominated by the loss of activation energy of the gel material. The Arrhenius acceleration model is widely used to predict the life, which expresses the life as a function of temperature. The Arrhenius formula is expressed as:

$$F = Ce^{-\frac{E}{kB*M}}$$

where F is the chemical reaction rate of gel material, and the life of the component can be calculated as the reciprocal of the chemical reaction rate of gel material, that is, 1/F; C represents the environmental variable, and for a gel material, its value is directly related to the sealing environment of components; e represents the natural logarithmic base; E represents the activation energy failure mechanism variable and will attenuate under changes in gas density; kB is the Boltzmann constant, specifically equal to $8.62 \times 10e^{\wedge}(-5e)$ V/K; M represents temperature.

According to the above formula, the activation energy of gel is directly affected by temperature, and its environmental variable C and activation energy failure mechanism variable E are also affected by the sealing environment: under the completely open environment without sealing, the effectiveness of gel material is constantly attenuated, and its activation energy will gradually fail within 24 hours.

Thus, in step S100, an environmental container for estimating electrochemical attenuation of a gel material is set, and for estimating the defibrillation electrode wear, environmental variables and activation energy failure mechanism variables is determined as the hidden parameters related to gas tightness, for electrochemical attenuation estimation of the gel material, wherein the environmental container is equipped with humidity sensors and gas pressure sensors for observing humidity and gas pressure sequences, and the environmental container is also equipped with a temperature sensor for observing temperature values.

In the process of establishing a hidden Markov model in step S200 of this detailed example, a Markov second order assumption is made, which means that the hidden state representation of the hyperparameters (environmental variables, activation energy failure mechanism variables) affected by gas tightness in the Arrhenius formula is only related to the previous hidden state and the observed gas pressure and humidity measurement values. The Markov model is established as shown in Fig.2. The specific steps are as follows:

S210, defining the tuples in the hidden Markov model:

S211, defining a set of humidity and gas pressure observation sequences, using the humidity and gas pressure observation sequences $\mathbf{O} = [o_1, o_2,..., o_T]$ of the timely measured humidity time sequence and gas pressure time sequence as the observation vectors of the hidden Markov chain, and defining a set of humidity and gas pressure observation sequences, wherein, T represents the period during which the humidity sensor and gas pressure sensor start to measure at a fixed time when the equipment is working, : $o_t = [pressure_t, humidity_t]^T$ is observation values of the gas pressure and humidity measured at time t.

S212, defining a set of hidden states, representing the gas-tightness hidden states as gas-tightness hidden state sequence Q of a Markov chain, and defining the gas-tightness hidden state sequence as $\mathbf{Q} = [q_1, q_2,..., q_T]$, wherein $q_t = [C_t, E_t]^T$ is the hidden representation of the hidden parameters related to gas tightness at the time t;

S213, defining a state probability matrix: for the state transition set of gas-tightness hidden states at different times, it is represented as the state probability matrix A, wherein each element $a_{ij}$ of the state probability matrix A represents the transition probability from state i to state j;

S214, defining an emission probability matrix: for the set of possible humidity and gas pressure measurement values generated in the gas-tightness hidden state, it is represented as the emission probability matrix B, wherein each elements $b_{qi}$ of the emission probability matrix B represents the emission probability from the hidden state $q_i$ to the observed state;

8215, defining the initial probability matrix: for the expression of the factory default of initial gas-tightness hidden state, it is represented as the initial probability matrix $\mathbf{\Pi}$, wherein each element $\pi_{qi}$ in the matrix represents the initial probability of the hidden state $q_i$, that is, the factory-default probability distribution;

S220, establishing a hidden Markov model, which is $\lambda = \{\mathbf{A}, \mathbf{B}, \mathbf{\Pi}\}$;

8230, performing hidden Markov process representation which is the output probability of the humidity and gas pressure observation sequence $\mathbf{O}$ in the model $\lambda = \{\mathbf{A}, \mathbf{B}, \mathbf{\Pi}\}$, wherein $P(O|\lambda)$ represents the joint probability of the humidity and gas pressure observation sequence $\mathbf{O}$ and the gas-tightness hidden state sequence $\mathbf{Q} = [q_1, q_2,..., q_T]$ occurring simultaneously in the model $\lambda$. The formula is as follows:

$$P(O|\lambda) = \sum_Q P(O|Q, \lambda) = \sum_{q_1, q_2,...q_T} \pi_{q_1} b_{q_1}(o_1) a_{q_1 q_2} b_{q_2}(o_2)...a_{q_{T-1} q_T} b_{q_T}(O_T)$$

For a fixed hidden state $\mathbf{Q} = [q_1, q_2,..., q_T]$, the probability of obtaining a humidity and gas pressure observation sequence $\mathbf{O} = [o_1, o_2,..., o_T]$ is $P(O|Q,\lambda) = b_{q_1}(o_1) b_{q_2}(o_2)...b_{q_T}(o_T)$.

In the process of learning for the hidden Markov model in step S300 of this detailed example, before the device leaves the factory, the defined Markov is learned for to obtain the optimal model parameters under the observation sequence $\mathbf{O}$, that is, a process to solve the optimal parameter set $\lambda = \{\mathbf{A}, \mathbf{B}, \mathbf{\Pi}\}$ which can maximize $P(\mathbf{O}|\lambda)$ with the given observation sequence $\mathbf{O}$. This can be performed by using Baum-Welch algorithm. The specific steps include:

S310, collecting K groups of humidity and gas pressure observation sequences $\mathbf{O} = [\mathbf{O}^1, \mathbf{O}^2,..., \mathbf{O}^K]$ , wherein

$\mathbf{O^k} = [O_1^k, O_2^k, \ldots, O_T^k]$ represents the $k^{th}$ group humidity and gas pressure observation sequence;

S320, obtaining the model parameters which makes the maximal joint probability value with the $k^{th}$ group humidity and gas pressure observation sequence $O^k$ and define the model parameters as optimal, the process is:

$$P(\mathbf{O}|\lambda) = \prod_{k=1}^K P(\mathbf{O^k}|\lambda) = \prod_{k=1}^K P_k \; ;$$

Wherein hidden Markov observations are continuous variables, and the probability density function of their emission probability is represented by a mixed one-dimensional Gaussian probability density function, as follows:

$$b_j\left(o_t^k\right) = \sum_{m=1}^{M} c_{jm} b_{jm}\left(o_t^k\right) = \sum_{m=1}^{M} c_{jm} \frac{1}{\sqrt{2\pi\sigma_{jm}^2}} \times \exp\frac{-\left(o_t^k - \mu_{jm}\right)^2}{2\sigma_{jm}^2};$$

wherein: $c_{jm}, \mu_{jm}, \sigma_{jm}$ represents the mixed weight, density, and variance of Gaussian density in $m^{th}$ column in the state $Q_j$, respectively. m=2 when the gas-tightness hidden state is only affected by humidity and gas pressure;

$\xi_t^k(i,j)$ is defined as a probability of being in a state $Q_i$ at time t and being in a state $Q_j$ at time t+1; $\xi_t^k(i)$ is defined as

a probability of being in a state $Q_i$ at time t, $\xi_{t,m}^k(i)$ is defined as a mixed probability in $m^{th}$ column of being in a state $Q_i$ at time t. The posterior probability of parameter estimation is expressed as follows:

$$\xi_{t,m}^k(i) = \frac{\alpha_t^k(i) a_{ij} b_j\left(o_{t+1}^k\right) \beta_{t+1}^k(j)}{\sum_{l=1}^{N} \alpha_t^k(l) \beta_t^k(l)};$$

$$\xi_t^k(i) = \frac{\alpha_t^k(i) \beta_{t+1}^k(j)}{\sum_{l=1}^{N} \alpha_t^k(l) \beta_t^k(l)};$$

$$\xi_{t,m}^k(i,j) = \frac{\alpha_t^k(i) \beta_{t+1}^k(i)}{\sum_{l=1}^{N} \alpha_t^k(l) \beta_t^k(l)} \times \frac{c_{im} b_{im}\left(o_t^k\right)}{\sum_{l=1}^{M} c_{il} b_{il}\left(o_t^k\right)};$$

where, $\alpha_t^k(i)$ and $\beta_t^k(j)$ are the forward and backward variables of the $k^{th}$ observation sample sequence.

S330, training by using K groups of humidity and gas pressure observation sequences to obtain the optimal parameter set with the given humidity and gas pressure observation sequence O;

S340, adjusting to make the joint probability of simultaneous occurrence of humidity and gas pressure observation sequences **O** and gas-tightness hidden state sequences **Q** = [$q_1$, $q_2$,..., $q_T$] under the model $\lambda$ maximum, and setting the optimal model parameters obtained with each humidity and gas pressure observation sequence as determined model parameters. The Baum-Welch algorithm is used to estimate the model parameters $\lambda$ = {A, B, $\pi$} as follows:

calculating the initial probability distribution $\Pi$ as follows:

$$\overline{\pi}_i = \frac{\sum_{k=1}^{K} \xi_1^k(i)}{K};$$

calculating the probability of state transition A as follows:

$$\overline{a}_{ij} = \frac{\sum_{k=1}^{K} \sum_{t=1}^{T-1} \xi_t^k(i,j)}{\sum_{k=1}^{K} \sum_{t=1}^{T-1} \xi_t^k(i)}$$

The mixed one-dimensional Gaussian probability density in the calculation of emission probability density B as follows:

$$\overline{a}_{jm} = \frac{\sum_{k=1}^{K} \sum_{t=1}^{T} \xi_{t,m}^k(j)}{\sum_{k=1}^{K} \sum_{t=1}^{T} \sum_{m=1}^{M} \xi_{t,m}^k(j)};$$

$$\overline{\mu}_{jm} = \frac{\sum_{k=1}^{K} \sum_{t=1}^{T} \xi_{t,m}^{k}(j) o_{t}^{k}}{\sum_{k=1}^{K} \sum_{t=1}^{T} \xi_{t,m}^{k}(j)};$$

$$\overline{\sigma}_{jm} = \frac{\sum_{k=1}^{K} \sum_{t=1}^{T} \xi_{t,m}^{k}(j) \left(o_{t}^{k} - \mu_{jm}\right)^{2}}{\sum_{k=1}^{K} \sum_{t=1}^{T} \xi_{t,m}^{k}(j)}.$$

In the process of decoding the hidden Markov model and estimating the defibrillation electrode wear in step S400 of this detailed example, the defined Markov is decoded to obtain the hidden representation of the gas tightness Q under the observation sequence O, that is, a process to solve the most likely state sequence Q under the given observation sequence O and the determined model parameters $\lambda = \{\mathbf{A}, \mathbf{B}, \mathbf{\Pi}\}$ that can maximize the joint probability $P(\mathbf{Q}|\lambda)$. **The specific steps are as follows:**

S410, among the determined model parameters, defining $\delta_t(i)$ as the maximal probability for generating $o_1, o_2,..., o_t$ along a path $q_1, q_2,..., q_T$ at time t;
S420, initializing $\delta_t(i) = \pi_i b_i(o_i)$;

S430, calculating the results $\delta_t(j) = \max_j (\delta_{t-1}(i) a_{ij} | b_j(o_t))$ of the humidity and gas pressure observation sequence corresponding to the previous humidity and gas pressure sequence on the next path j;

S440, taking the maximum value in the step S430 path as the output result $P(\mathbf{Q}|\lambda) = \max_j (\delta_{t-1}(i) a_{ij})$, wherein $P(\mathbf{Q}|\lambda)$ is the parameter represented by the environmental variable C and the activation energy failure mechanism variable E under the influence of gas tightness.

S450, using the environmental variable C and the activation energy failure mechanism variable E obtained in step S440, as well as the temperature value measured by the temperature sensor, the chemical reaction rate is estimated using the reaction formula $F = C e^{-\frac{E}{kB*M}}$. The life of the defibrillation electrode can be calculated as the reciprocal of the chemical reaction rate of the gel material, that is, 1/F.

The validity period of the electrode is Y years. Due to changes in the environment, the F value also changes. After algorithm analysis, the F value can be obtained and validity period YT=Y/F, such that current validity period of the electrode can be obtained. The calculation results can be displayed through the display module of the detection module, or displayed on the defibrillator, the rescuer's mobile end, or the emergency platform end, or on at least one of the above.

Second embodiment

The second embodiment of the present invention proposes a defibrillation electrode assembly, which can intelligently display defibrillation electrode wear. The assembly includes an environment container, defibrillation electrodes, an island electrode, and a detection module. The environment container can be a sealed environment container, which is equipped with a humidity sensor for observing humidity sequences, a gas pressure sensor for observing gas pressure sequences, and a temperature sensor for observing temperature values. The humidity sensor, the gas pressure sensor, and the temperature sensor are connected to the detection module. The detection module estimates the defibrillation electrode wear based on the defibrillation electrode wear estimation method described in the first aspect of the present invention using parameters obtained from the humidity sensor, the gas pressure sensor, and the temperature sensor. The estimation result is displayed by a display module.

In the second embodiment of the present invention, the defibrillation electrodes and the island electrode are located in the same environmental container, the two defibrillation electrodes are connected to an electrode plug through a defibrillation discharging high-voltage line, and the island electrode is connected to the detection module, which is used to monitor various parameters of the island electrode. The detection module is inserted into a defibrillator through the electrode plug, and a detection module controller obtains various parameters sent from the sensors, and then performs algorithm analysis or directly reads the performance parameters of the defibrillation electrodes. Temperature, gas pressure, tightness and gel impedance are performance parameters to judge the effectiveness of the defibrillation electrode.

In a preferred embodiment of the present invention, the island electrode contains two test sheets with the same material as that of the defibrillation electrodes, that is, except for the size, the structure and material of the test sheet are the same as those of the defibrillation electrodes, so the monitored gel impedance parameters of the island electrode can be completely equivalent to the gel impedance test value of the defibrillation electrode after conversion of size differences.

The implementation method of gel impedance detection is to superimpose oppositely the defibrillation layers of the test sheets and a non-adhesive paper. The detection module includes: impedance test points set on the defibrillation layer of the test sheet. The impedance test points of the two test sheets are connected to the detection module through an impedance detection line to form an impedance detection circuit for inputted direct current. After inputted direct current, analog signals can be calculated according to the voltage and current data, and then the impedance of the gel layer to be obtained can be calculated from the detected analog signals. This DC impedance detection method is easy to achieve and the results are more accurate.

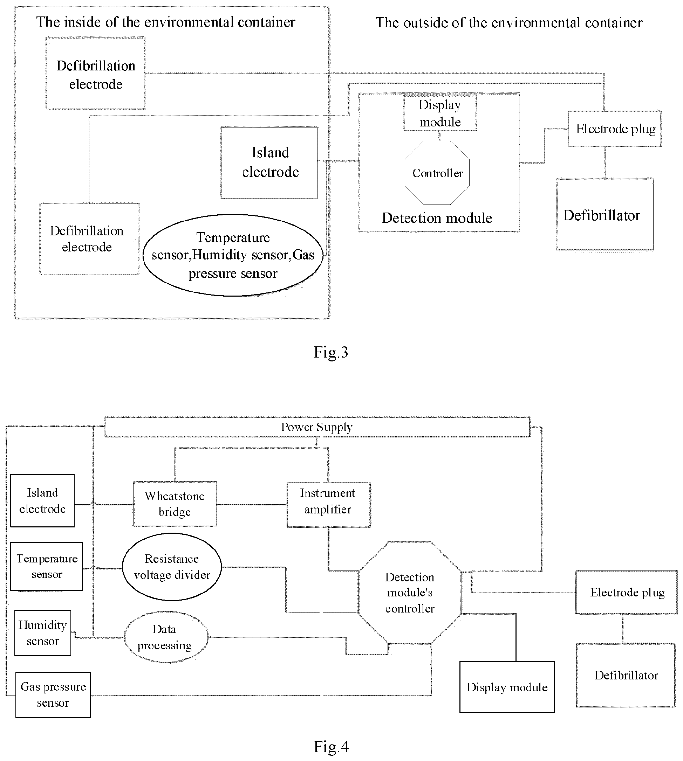

The detection module also includes a temperature sensor detection circuit and/or a humidity sensor detection circuit and/or a gas pressure sensor detection circuit/display screen. Each sensor detection circuit and display screen are respectively connected to the detection module controller through their respective connecting wires. Specifically, the temperature sensor detection circuit detects the detection signal through a temperature detection line and is then connected to the detection module controller. The humidity sensor detection circuit is connected to the detection module through a humidity detection line, and then connected to the detection module controller; the gas pressure sensor is connected to the detection module through a gas pressure detection line, and then connected to the detection module controller. It should be understood that the detection lines in this article are designed with input and output lines to form a detection loop, that is, the detection lines as shown in Fig. 3 are multiple lines.

The reason for using the temperature sensor is that the internal temperature of the defibrillation electrode will directly affect the adhesion of the gel to the human body, and too high or too low temperature will even directly lead to the failure of the electrode. Therefore, the temperature of the defibrillation electrode can effectively reflect the effectiveness of the defibrillation electrode.

The reason for using humidity sensor and gas pressure sensor is that defibrillation electrode is usually sealed in environmental container. In an unsealed state, the defibrillation electrode usually has a validity period of only 24 hours. Therefore, the tightness of defibrillation electrode packaging can effectively reflect the effectiveness of defibrillation electrode. The changes in humidity and gas pressure in the sealed environment can determine whether there is any leakage or other faults in the sealing of the environmental container, that is, the tightness can be reflected by the humidity and gas pressure information inside the environmental container.

The reason for using the display screen is that the performance parameters of the defibrillation electrodes can be displayed on the display screen, or on a displayer or the cloud of the defibrillator. Such display would be intuitive and clear.

As shown in Figs 3 and 4, in some embodiment of the present invention, the detection module is connected to the temperature sensor, the humidity sensor, and the gas pressure sensor. The detection module is equipped with a controller and a display screen. The humidity sensor is connected to an IIC communication channel of the controller through a data processing module, and the temperature sensor is connected in series with a second resistor and then connected to a second ADC channel of the controller. The temperature AD value is detected through the second ADC channel, and the humidity AD value is detected through the IIC communication channel. The controller can then obtain temperature and humidity information based on a temperature and humidity impedance linear table. The impedance test point is connected in series with an impedance test bridge and an instrument amplifier and then connected a third ADC channel of the controller, and the controller converts the AD value of the third ADC channel to calculate the gel impedance information. The gas pressure sensor is connected to a serial communication channel of the controller. The gas pressure sensor converts the changes in gas pressure into electrical signals. After amplified, the electrical signals are converted into digital signals from analog signals by AD conversion, and finally transmitted to the controller through serial communication. The controller communicates with the display screen through SPI communication to display the parameters of the defibrillation electrode.

In some embodiment of the present invention, the temperature sensor adopts a thermistor, which is built-in in the environmental container or the detection module to improve the accuracy of temperature monitoring.

It should be understood that due to the fact that the island electrode and defibrillation electrode are located in the same environmental container, that is, both of them are in the same environment, and the material of the island electrode is the same as that of the defibrillation electrode, the internal temperature of the island electrode can be equivalent to the internal temperature of the defibrillation electrode.

As shown in Fig.3, the island electrode and/or humidity sensor and/or gas pressure sensor and/or temperature sensor are installed inside the environmental container. A preferred solution is to have the island electrode, the humidity sensor, the temperature sensor, and the pressure sensor adjacent to each other, so that the island electrode, temperature sensor, humidity sensor, and gas pressure sensor are completely independent of and outside the defibrillation electrode. The defibrillation electrode is only connected to high voltage wires for defibrillation discharging, thus wires are less and the electrode is light and thin. Moreover, since no additional sensor is installed on the defibrillation electrode, the interference during defibrillation operation is minimal, effectively improving the defibrillation effect.

As shown in Fig.3, the defibrillator is located outside the environmental container, and the detection module is located between the environmental container and the defibrillator. The detection lines extend from the environmental container and are connected to the detection module. The electrode plug is connected to the defibrillator electrode and the detection module, and the detection module is connected to the island electrode and various sensors. The electrode plug is inserted into the interior of the defibrillator. It should be understood that the "connection" mentioned in this article can be a wired connection, such as the various detection lines mentioned above, or a wireless connection. There is no special limitations on the implementation of the connection and it is sufficient for the connection to achieve energy or signal transmission.

On this basis, the area of the island electrode is smaller than that of the defibrillator electrode, reducing the space occupied by the island electrode and production costs. The defibrillator is located on one side of the environmental container, and the island electrode is set close to the detection module. The detection module is located between the island electrode and the defibrillator, shortening the length of the detection line between the island electrode and the detection module.

The third embodiment

The third embodiment of the present invention further proposes a defibrillation device with the aforementioned defibrillation electrode assembly. The defibrillation device includes a defibrillator. The defibrillator has a controller inside the defibrillator, which obtains real-time temperature, humidity, packaging tightness, impedance data of the defibrillation electrode through the detection module, and makes judgments on the effectiveness and defibrillation electrode wear. The controller of the detection module can also obtain real-time temperature, humidity, packaging tightness, impedance data of the defibrillation electrode, and estimate the defibrillation electrode wear. Relevant personnel is notified through the detection module's display screen, defibrillator's display screen, defibrillator cloud networking, voice broadcasting, or other means.

The fourth embodiment

The fourth embodiment of the present invention additionally proposes a readable storage medium on which a control program is stored. When the control program is executed by the detection module, the detection module can execute the steps of the method for estimating the defibrillation electrode wear as described in the first embodiment of the present invention.

The fifth Embodiment

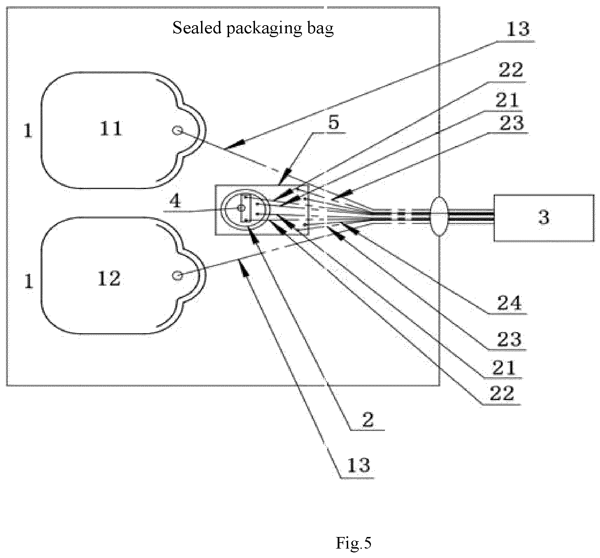

In the fifth Embodiment, the present invention, as shown in Fig.5, proposes another kind of defibrillation electrode assembly including two defibrillation electrodes 1 and an island electrode 2. The two defibrillation electrodes 1 are a left defibrillation electrode 11 and a right defibrillation electrode 12. The defibrillation electrode 1 has a defibrillation layer, the defibrillation layer comprises a conductive layer and a gel layer arranged on the outer surface of the conductive layer. The outer surface of the gel layer is covered with non-adhesive paper, which is used to protect the gel layer.

The defibrillation electrode 1 and island electrode 2 are located in the same sealed packaging bag. The two defibrillation electrodes 1 are connected to an external connection terminal 3 through a defibrillation discharging high-voltage line 13. The island electrode 2 is equipped with a detection module, which is used to monitor various parameters of island electrode 2. The detection module is connected to the external connection terminal 3. A controller obtains various parameters sent by the detection module from the external connection terminal 3, and then calculates or directly reads the performance parameters of the defibrillation electrode. Temperature, gas pressure, tightness and gel impedance are performance parameters to judge the effectiveness of the defibrillation electrode.

In the embodiment of the present invention, the island electrode 2 includes two test sheets with the same material as that of the defibrillation electrode 1, that is, except for the size, the structure and material of the test sheet are the same as those of the defibrillation electrode, so the monitored gel impedance parameters of the island electrode 2 can be completely equivalent to the gel impedance test value of the defibrillation electrode 1 after conversion of the size difference.

The realization method of gel impedance detection is to stack oppositely the defibrillation layers of the two test sheets. The detection module includes impedance test points set on the defibrillation layer of the test sheet. The impedance test points of the two test sheets are connected to the external connection terminal 3 through an impedance detection line 21, respectively, to form an impedance detection circuit for inputted alternating current. The two test sheets of the island electrode 2 are stacked to form a capacitor of the impedance detection circuit. After alternating current is inputted, the capacitance of the capacitor can be calculated according to the voltage and current data, and then the impedance of the gel layer to be obtained can be calculated from the detected capacitance. This way of alternating current impedance detection is easy to achieve, and the results are more accurate.

The detection module also includes a temperature sensor 4 and/or a humidity sensor 5 and/or a gas pressure sensor. Each sensor is connected to the external connection end through its corresponding detection line. Furthemore, temperature sensor 4 is connected to the external connection end 3 through a temperature detection line 22, humidity sensor 5 is connected to the external connection end 3 through a humidity detection line 23, and the gas pressure sensor is connected to the external connection end 3 through a gas pressure detection line 24. It should be understood that the detection lines in this article are designed having input and output lines to form a detection loop, that is, each type of detection line as shown in Fig.5 has two lines.

The reason for using temperature sensor 4 is that the internal temperature of the defibrillation electrode 1 will directly affect the adhesion of the gel to the human body, and too high or too low temperature will even directly lead to the failure of the electrode. Therefore, the temperature of the defibrillation electrode 1 can effectively reflect the effectiveness of the defibrillation electrode 1.

The reason for using the humidity sensor 5 and the gas pressure sensor is that defibrillation electrode 1 is usually sealed in a packaging bag. In an unsealed state, defibrillation electrode 1 usually has a validity period of only 24 hours. Therefore, the tightness of defibrillation electrode 1 packaging can effectively reflect the effectiveness of defibrillation electrode 1. The humidity and gas pressure changes in the sealed environment can determine whether there is any leakage or other faults in the packaging bag's sealing, that is, the tightness can be reflected by the humidity and gas pressure information inside the packaging bag.

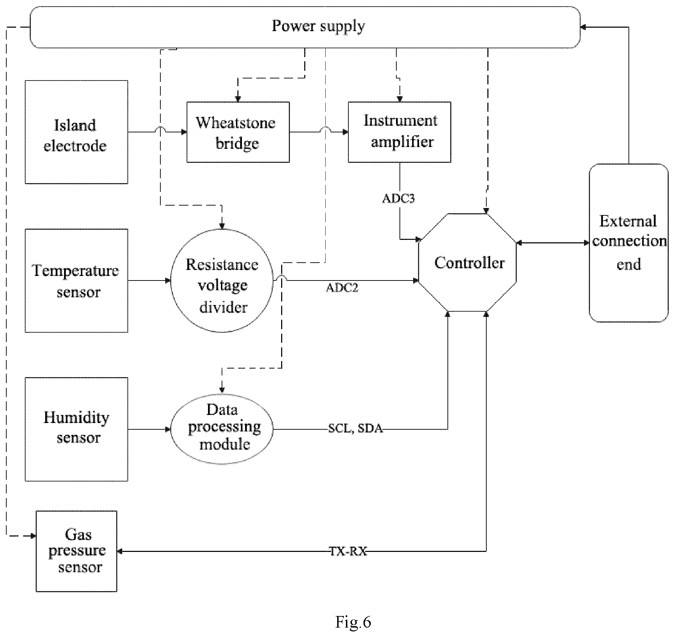

As shown in Figs 5 and 6, in some embodiment of the present invention, the detection module includes a temperature sensor 4, a humidity sensor 5, and a gas pressure sensor. An external connection end 3 is connected to a controller. The humidity sensor is connected to an IIC communication channel of the controller through a data processing module. The temperature sensor 4 is connected in series with a second resistor and then connected to a second ADC channel of the controller. The temperature AD value is detected through the second ADC channel. The humidity AD value is detected through the IIC communication channel. The controller can obtain temperature and humidity information based on a temperature and humidity impedance linear table. The impedance test point is connected in series with an impedance test bridge and an instrument amplifier and then connected to a third ADC channel of the controller. The controller converts the AD value of the third ADC channel to calculate the gel impedance information. The gas pressure sensor is connected to a serial communication channel of the controller. The gas pressure sensor converts the change in gas pressure into an electrical signal. After amplified, the electrical signal is converted into a digital signal through AD conversion from analog signal, and finally transmitted to the controller through serial communication.

In some embodiment of the present invention, the temperature sensor 4 adopts a thermistor, which is embedded in any test sheet of the island electrode 2 to improve the accuracy of temperature monitoring.

It should be understood that due to the fact that the island electrode 2 and defibrillation electrode 1 are located in the same sealed packaging bag, that is, they are in the same environment, and the material of island electrode 2 is the same as that of defibrillation electrode 1, the internal temperature of island electrode 2 can be equivalent to that of defibrillation electrode 1.

As shown in Fig.5, the island electrode 2 is installed on the humidity sensor 5 and/or the gas pressure sensor. The optional solution is to have the humidity sensor 5 and the gas pressure sensor adjacent to each other. The island electrode 2 is installed on the humidity sensor 5 and the gas pressure sensor, so that the island electrode plate 2, the temperature sensor 4, the humidity sensor 5, and the gas pressure sensor are completely independent of and outside the defibrillation electrode 1 as a whole. The defibrillation electrode 1 is designed only to connect with a high-voltage defibrillation discharging line 13. Wiring is less and the electrode is light and thin. Moreover, since the defibrillation electrode 1 does not have an additional sensor installed thereon, the interference during defibrillation operation is minimal, effectively improving the defibrillation effect.

As shown in Fig. 5, the external connection end 3 is located outside the sealed packaging bag, and the detection lines extend from inside the sealed packaging bag and are connected to the external connection end 3. The controller or other upper or host computer connects the defibrillation electrode 1 and the island electrode 2 through the external connection end 3. It should be understood that the "connection" mentioned in this article can be a wired connection, such as the various detection lines mentioned above, or a wireless connection. There are no special limitations on the implementation of the connection in the present invention and it is sufficient for the connection to achieve energy or signal transmission.

On this basis, the area of the island electrode 2 is smaller than that of the defibrillation electrode 1, reducing the space occupied by the island electrode 2 and lowering production costs. The external connection end 3 is located on one side of the sealed packaging bag, and the island electrode 2 is set close to the external connection end 3 to shorten the length of the detection line between the island electrode 2 and the external connection end 3.

The present invention also proposes a defibrillation device with the above-mentioned defibrillation electrode assembly. The external connection end 3 is connected to a controller of the defibrillation device. The controller obtains real-time temperature, humidity, packaging tightness, and impedance data of the defibrillation electrode 1 through the external connection end, and makes judgments on the effectiveness of the defibrillation electrode 1. The controller then notifies relevant personnel through networking, voice broadcasting, or other means.

Compared with existing technology, the present invention can effectively monitor the status of defibrillation electrodes while maintaining their working performance. It not only greatly reduces the cost of manual maintenance and monitoring, but also has high monitoring accuracy and good defibrillation effect.

It should be noted that the terms used above are only intended to describe specific embodiment and are not intended to limit exemplary embodiment according to the present invention. As used here, unless otherwise explicitly stated in the context, the singular form is also intended to include the plural form. In addition, it should be understood that when the terms "including" and/or "comprising" are used in this specification, they indicate the existence of features, steps, operations, devices, components, and/or their combinations.

## Claims

1. A method for estimating defibrillation electrode wear, comprising:

   S100, setting an environmental container for estimating electrochemical attenuation of a gel material of the defibrillation electrode, and determining environmental variables and activation energy failure mechanism variables as hidden parameters related to gas tightness, for estimating electrochemical attenuation of the gel material of the defibrillation electrode, so as to estimate defibrillation electrode wear, wherein the environmental container is equipped with a humidity sensor and a gas pressure sensor, for observing humidity and gas pressure sequences;

   S200, establishing a hidden Markov model, and using it to establish a state transition process for hidden parameters related to gas tightness, under the observed humidity and gas pressure sequences, wherein the observed values of humidity and gas pressure sequences are used as observation vectors of hidden Markov chain and are defined as a set of humidity and gas pressure observation sequences, and the hidden parameters related to gas tightness are used as hidden state vectors of the hidden Markov chain and are defined as gas-tightness hidden state sequences;

   S300, learning for the hidden Markov model and adjusting parameters of the model using collected multiple sets of humidity and gas pressure observation sequences, wherein a set of optimal model parameters obtained under each humidity and gas pressure observation sequences is determined as determined model parameters;

   S400, decoding the hidden Markov model and estimating the defibrillation electrode wear, wherein when using a defibrillation device, the decoding is based on the determined model parameters and the humidity and gas pressure observation sequences corresponding to the humidity and gas pressure sequences at a previous moment, so as to obtain hidden parameters related to gas tightness corresponding to the current gas-tightness hidden state sequence, wherein the environmental variables and activation energy failure mechanism variables corresponding to the hidden parameters related to gas tightness are used to estimate the life measurement of the gel material of the defibrillation electrode, and an estimated current value of the defibrillation electrode wear is finally obtained and displayed.

2. The method for estimating defibrillation electrode wear according to claim 1, wherein in step S100, the environmental container is additionally equipped with a temperature sensor for observing temperature values and wherein in step S400, the value of defibrillation electrode wear is estimated additionally based on the temperature values.

3. The method for estimating defibrillation electrode wear according to claim 2, wherein the following reaction formula is used to estimate life loss of the electrode mainly based on the loss of activation energy of gel material of the defibrillation electrode:

$$F = Ce^{-\frac{E}{kB*M}}$$

   where F is chemical reaction rate of gel material of the defibrillation electrode, and the life of the defibrillation electrode can be calculated as the reciprocal of the chemical reaction rate of gel material of the defibrillation electrode, that is, 1/F; C represents the environmental variable; e represents the natural logarithmic base; E represents the activation energy failure mechanism variable; kB is Boltzmann constant; M represents temperature.

4. The method for estimating defibrillation electrode wear according to claim 3, wherein the step S200 comprising:

   S210, defining each tuple in the hidden Markov model, wherein each tuple comprises a set of humidity and gas pressure observation sequences, a hidden state set, a state probability matrix, an emission probability matrix, and an initial probability matrix;

S220, establishing a hidden Markov model, which is related to the state probability matrix, the emission probability matrix, and the initial probability matrix;

S230, performing a hidden Markov process representation, wherein the hidden Markov process representation is an output probability of the humidity and gas pressure observation sequences under the hidden Markov model, i.e. a joint probability that the humidity and gas pressure observation sequence and the gas-tightness hidden state representation occur simultaneously under the hidden Markov model.

5. The method for estimating defibrillation electrode wear according to claim 4, wherein the step S200 comprising:

S210, defining the tuples in the hidden Markov model,

S211, defining a set of humidity and gas pressure observation sequences, using the humidity and gas pressure observation sequences $O = [o_1, o_2,..., o_T]$ of the timely measured humidity time sequence and gas pressure time sequence as observation vector of hidden Markov chain, and defining a set of humidity and gas pressure observation sequences, where T represents a period during which the humidity sensor and the gas pressure sensor start to measure at a fixed time when the defibrillation device is working, $o_t = [pressure_t, humidity_t]^T$ is observation values of the gas pressure and humidity measured at time t;

S212, defining a set of hidden states, taking the gas-tightness hidden state representation as a gas-tightness hidden state sequence Q of the Markov chain, and defining the gas-tightness hidden state sequence as $Q = [q_1, q_2,..., q_T]$, wherein $qt = [C_t, E_t]^T$ is the hidden representation of the hidden parameters related to gas tightness at time t;

S213, defining a state probability matrix, wherein for the state transition set of gas-tightness hidden states at different times, it is represented as a state probability matrix A, wherein each element $a_{ij}$ of the state probability matrix A represents the transition probability from state i to state j;

S214, defining an emission probability matrix: for the set of possible humidity and gas pressure measurement values generated in the gas-tightness hidden state, it is represented as an emission probability matrix B, wherein the element $b_{qi}$ of the emission probability matrix B represents the emission probability from a hidden state $q_i$ to an observation state;

S215, defining an initial probability matrix: for the expression of the factory default of initial gas-tightness hidden state, it is represented as an initial probability matrix $\Pi$, wherein each element $\pi_{qi}$ in the matrix represents initial probability of the hidden state $q_j$, that is, the factory-default probability distribution;

S220, establishing a hidden Markov model, which is $\lambda = \{A, B, \Pi\}$;

S230, performing a hidden Markov process representation, wherein the hidden Markov process representation is the output probability of the humidity and gas pressure observation sequence $O$ under the model $\lambda = \{A, B, \Pi\}$, wherein $P(O|\lambda)$ represents a joint probability that the humidity and gas pressure observation sequence O and the gas-tightness hidden state sequence $Q = [q_1, q_2,..., q_T]$ occur simultaneously under the model $\lambda$, wherein the formula is as follows:

$$P(O|\lambda) = \sum_Q P(O|Q,\lambda) = \sum_{q_1,q_2...q_T} \pi_{q_1} b_{q_1}(o_1) a_{q_1q_2} b_{q_2}(o_2)...a_{q_{T-1}q_T} b_{q_T}(O_T)$$

for a fixed hidden state $Q = [q_1, q_2,..., q_T]$, the probability of obtaining a humidity and gas pressure observation sequence $O = [o_1, o_2,..., o_T]$ is $P(O|Q, \lambda) = b_{q_1}(o_1)b_{q_2}(o_2)...b_{q_T}(O_T)$.

6. The method for estimating defibrillation electrode wear according to claim 5, wherein the step S300 of learning for the hidden Markov model comprising:

S310, collecting K groups of humidity and gas pressure observation sequences $O = [O^1, O^2,..., O^K]$, wherein

$$O^k = [O_1^k, O_2^k, \ldots, O_T^k]$$ represents the $k^{th}$ group humidity and gas pressure observation sequence;

S320, obtaining the model parameters which makes the maximal joint probability value under the $k^{th}$ group humidity and gas pressure observation sequence $O^k$, and defining the model parameters as optimal;

S330, training by using K groups of humidity and gas pressure observation sequences to obtain the optimal parameter set with the given humidity and gas pressure observation sequence O;

S340, by adjusting, making the joint probability that the humidity and gas pressure observation sequence $O$ and the gas-tightness hidden state sequence $Q = [q_1, q_2,..., q_T]$ occur simultaneously under the model $\lambda$ to be maximum, and setting the optimal model parameters obtained with each humidity and gas pressure observation sequence as determined model parameters.

7. The method for estimating defibrillation electrode wear according to claim 6, wherein in step S400, the step for obtaining hidden parameters related to gas tightness corresponding to the gas-tightness hidden state sequence comprising:

S410, among the detennined model parameters, defining $\delta_t(i)$ as maximum probability for generating $o_1, o_2,..., o_t$ along a path $q_1, q_2,..., q_T$ at time t;

S420, initializing $\delta_t(i) = \pi_i b_i(o_i)$;

S430, calculating the results $\delta_t(j) = \max_j (\delta_{t-1}(i)a_{ij}|b_j(o_t))$ of the humidity and gas pressure observation sequence corresponding to the previous humidity and gas pressure sequence on the next path j;

S440, taking the maximum value in the path in the step S430 as the output result

$$P(\mathbf{Q}|\lambda) = \max_j (\delta_{t-1}(i)a_{ij})$$, wherein $P(\mathbf{Q}|\lambda)$ is the parameter represented by the environmental variable C and the activation energy failure mechanism variable E under the influence of gas tightness.

8. A defibrillation electrode assembly for intelligent display of defibrillation electrode wear, wherein the assembly comprises an environmental container, a defibrillation electrode, an island electrode, and a detection module, wherein the defibrillation electrode and the island electrode are placed inside the environmental container, and the detection module comprises a humidity sensor, a gas pressure sensor, and a temperature sensor placed in the environmental container, wherein the detection module is configured to estimate the defibrillation electrode wear by carrying out the method for estimating defibrillation electrode wear according to any one of claims 1 to 7 using parameters obtained from the humidity sensor, pressure sensor, and temperature sensor, and is configured to display the estimation result through a display module.

9. The defibrillation electrode assembly according to claim 8, wherein the detection module comprises a controller, the humidity sensor is connected to the controller through a data processing module, the temperature sensor is connected in series with a resistance voltage divider module and connected to the controller, and the gas pressure sensor is connected to the controller,
preferably, the island electrode comprises two test sheets of the same material as that of the defibrillation electrode, the defibrillation layers of the two test sheets are stacked oppositely, and the impedance test points of the two test sheets are connected to the detection module to form an impedance detection circuit for inputted direct current.

10. A readable storage medium, wherein a control program is stored on the readable storage medium, and the control program enables, when executed by the detection module of the defibrillation electrode assembly according to claim 8 or claim 9, the detection module to execute the steps of the method as claimed in any one of claims 1 to 7.

11. The defibrillation electrode assembly of claim 8 or 9, which comprises two defibrillation electrodes located in a sealed packaging bag,

wherein the two defibrillation electrodes are connected to an external connection end through a defibrillation discharging high-voltage wire;
wherein the island electrode is used for feedback on the performance parameters of the defibrillation electrode, the island electrode is placed in the sealed packaging bag, and the island electrode is equipped with the detection module, which is connected to the external connection end.

12. The defibrillation electrode assembly according to claim 11, wherein the island electrode comprises two test sheets of the same material as that of the defibrillation electrode, and the defibrillation layers of the two test sheets are overlapped oppositely;
wherein the detection module comprises an impedance test point located on the defibrillation layer, and the impedance test points of the two test sheets are connected to the external connection end to form an impedance detection circuit for inputted alternating current.

13. The defibrillation electrode assembly according to claim 12, wherein the detection module is connected to the external connection end through a detection wire, the external connection end is located on the outside of the sealed packaging bag, and the detection wire extends from the sealed packaging bag and is connected to the external connection end.

**14.** The defibrillation electrode assembly according to claim 13, wherein the area of the island electrode is smaller than that of the defibrillation electrode,

preferably, the external connection end is located on one side of the sealed packaging bag, and the island electrode piece is arranged near the external connection end.

**15.** A defibrillation device, wherein the defibrillation device has a defibrillation electrode assembly according to any one of claims 8 to 14, and a defibrillator electrically connected to the defibrillation electrode assembly.

**Patentansprüche**

**1.** Verfahren zum Schätzen eines Defibrillationselektrodenverschleißes, umfassend:

S100, Einstellen eines Klimabehältnisses zum Schätzen einer elektrochemischen Abschwächung eines Gelmaterials der Defibrillationselektrode und Bestimmen von Klimavariablen und Aktivierungsenergieausfallmechanismusvariablen als verborgene Parameter, die sich auf die Gasdichtheit beziehen, zum Schätzen der elektrochemischen Abschwächung des Gelmaterials der Defibrillationselektrode, um einen Verschleiß der Defibrillationselektrode zu schätzen, wobei das Klimabehältnis mit einem Feuchtigkeitssensor und einem Gasdrucksensor zum Beobachten von Feuchtigkeits- und Gasdrucksequenzen ausgestattet ist;

S200, Einrichten eines Hidden-Markow-Modells und Verwenden desselben, um einen Zustandsübergangsprozess für verborgene Parameter in Bezug auf Gasdichtheit unter den beobachteten Feuchtigkeits- und Gasdrucksequenzen einzurichten, wobei die beobachteten Werte von Feuchtigkeits- und Gasdrucksequenzen als Beobachtungsvektoren einer Hidden-Markow-Kette verwendet werden und als ein Satz von Feuchtigkeits- und Gasdruckbeobachtungssequenzen definiert sind und die verborgenen Parameter in Bezug auf Gasdichtheit als verborgene Zustandsvektoren der Hidden-Markow-Kette verwendet werden und als verborgene Gasdichtheitszustandssequenzen definiert sind;

S300, Lernen für das Hidden-Markow-Modell und Anpassen von Parametern des Modells unter Verwendung vielfacher gesammelter Sätze von Feuchtigkeits- und Gasdruckbeobachtungssequenzen, wobei ein Satz von optimalen Modellparametern, die unter jeder Feuchtigkeits- und Gasdruckbeobachtungssequenz erhalten werden, als bestimmte Modellparameter bestimmt ist;

S400, Decodieren des Hidden-Markow-Modells und Schätzen des Defibrillationselektrodenverschleißes, wobei, wenn eine Defibrillationsvorrichtung verwendet wird, das Decodieren auf den bestimmten Modellparametern und den Feuchtigkeits- und Gasdruckbeobachtungssequenzen basiert, die den Feuchtigkeits- und Gasdrucksequenzen zu einem früheren Zeitpunkt entsprechen, um verborgene Parameter in Bezug auf Gasdichtheit zu erhalten, die der aktuellen verborgenen Gasdichtheitszustandssequenz entsprechen, wobei die Klimavariablen und Aktivierungsenergieausfallmechanismusvariablen, die den verborgenen Parametern in Bezug auf Gasdichtheit entsprechen, verwendet werden, um die Lebensdauermessung des Gelmaterials der Defibrillationselektrode zu schätzen, und ein geschätzter aktueller Wert des Defibrillationselektrodenverschleißes schließlich erhalten und angezeigt wird.

**2.** Verfahren zum Schätzen von Defibrillationselektrodenverschleiß nach Anspruch 1, wobei in Schritt S100 das Klimabehältnis zusätzlich mit einem Temperatursensor zum Beobachten von Temperaturwerten ausgestattet ist und wobei in Schritt S400 der Wert eines Defibrillationselektrodenverschleißes zusätzlich basierend auf den Temperaturwerten geschätzt wird.

**3.** Verfahren zum Schätzen von Defibrillationselektrodenverschleiß nach Anspruch 2, wobei die folgende Reaktionsformel verwendet wird, um einen Lebensdauerverlust der Elektrode hauptsächlich basierend auf dem Verlust von Aktivierungsenergie des Gelmaterials der Defibrillationselektrode zu schätzen:

$$F = C\, e^{-\frac{E}{kB*M}}$$

,

wobei F eine chemische Reaktionsrate des Gelmaterials der Defibrillationselektrode ist und die Lebensdauer der Defibrillationselektrode als der Kehrwert der chemischen Reaktionsrate des Gelmaterials der Defibrillationselektrode berechnet werden kann, d. h. 1/F; C die Klimavariable darstellt; e die Basis des natürlichen Logarithmus darstellt; E die Aktivierungsenergieausfallmechanismusvariable darstellt; kB eine Boltzmann-Konstante ist; M eine Temperatur darstellt.

4. Verfahren zum Schätzen von Defibrillationselektrodenverschleiß nach Anspruch 3, wobei der Schritt S200 Folgendes umfasst:

S210, Definieren jedes Tupels in dem Hidden-Markow-Modell, wobei jedes Tupel einen Satz von Feuchtigkeits- und Gasdruckbeobachtungssequenzen, einen verborgenen Zustandssatz, eine Zustandswahrscheinlichkeitsmatrix, eine Emissionswahrscheinlichkeitsmatrix und eine Anfangswahrscheinlichkeitsmatrix umfasst;

S220, Einrichten eines Hidden-Markow-Modells, das in Bezug mit der Zustandswahrscheinlichkeitsmatrix, der Emissionswahrscheinlichkeitsmatrix und der Anfangswahrscheinlichkeitsmatrix steht;

S230, Durchführen einer Hidden-Markow-Prozessdarstellung, wobei die Hidden-Markow-Prozessdarstellung eine Ausgabewahrscheinlichkeit der Feuchtigkeits- und Gasdruckbeobachtungssequenzen nach dem Hidden- Markow-Modell ist, d. h. eine Verbundwahrscheinlichkeit, dass die Feuchtigkeits- und Gasdruckbeobachtungssequenz und die verborgene Gasdichtheitszustandsdarstellung nach dem Hidden-Markow-Modell gleichzeitig auftreten.

5. Verfahren zum Schätzen von Defibrillationselektrodenverschleiß nach Anspruch 4, wobei der Schritt S200 Folgendes umfasst:

S210, Definieren der Tupel in dem Hidden-Markow-Modell;

S211, Definieren eines Satzes von Feuchtigkeits- und Gasdruckbeobachtungssequenzen unter Verwendung der Feuchtigkeits- und Gasdruckbeobachtungssequenzen $O = [o_1, o_2,..., o_T]$ der zeitlich gemessenen Feuchtigkeitszeitsequenz und Gasdruckzeitsequenz als Beobachtungsvektor der Hidden-Markow-Kette und Definieren eines Satzes von Feuchtigkeits- und Gasdruckbeobachtungssequenzen, wobei T einen Zeitraum darstellt, während dessen der Feuchtigkeitssensor und der Gasdrucksensor beginnen, zu einem festen Zeitpunkt, wenn die Defibrillationsvorrichtung arbeitet, zu messen, $o_t = [Druckt, Feuchtigkeit_t]^T$ Beobachtungswerte des Gas- drucks und der Feuchtigkeit ist, die zum Zeitpunkt t gemessen werden;

S212, Definieren eines Satzes von verborgenen Zuständen unter Hinzuziehung der verborgenen Gasdichtheitszustandsdarstellung als eine verborgene Gasdichtheitszustandssequenz Q der Markow-Kette und Definieren der verborgenen Gasdichtheitszustandssequenz als $Q = [q_1, q_2,..., q_T]$, wobei $qt = [Ct, E_t]^T$ die verborgene Darstellung der verborgenen Parameter in Bezug auf Gasdichtheit zum Zeitpunkt t ist;

S213, Definieren einer Zustandswahrscheinlichkeitsmatrix, wobei sie für den Zustandsübergangssatz von verborgenen Gasdichtheitszuständen zu verschiedenen Zeitpunkten als eine Zustandswahrscheinlichkeitsmatrix A dargestellt ist, wobei jedes Element $a_{ij}$ der Zustandswahrscheinlichkeitsmatrix A die Übergangswahrscheinlichkeit von Zustand i zu Zustand j darstellt;

S214, Definieren einer Emissionswahrscheinlichkeitsmatrix: für den Satz möglicher Feuchtigkeits- und Gasdruckmesswerte, die in dem verborgenen Gasdichtheitszustand erzeugt werden, wird sie als eine Emissionswahrscheinlichkeitsmatrix B dargestellt, wobei das Element $b_{qi}$ der Emissionswahrscheinlichkeitsmatrix B die Emissionswahrscheinlichkeit von einem verborgenen Zustand $q_i$ in einen Beobachtungszustand darstellt;

S215, Definieren einer Anfangswahrscheinlichkeitsmatrix: für den Ausdruck der Werkseinstellung eines verborgenen Anfangsgasdichtheitszustands wird sie als eine Anfangswahrscheinlichkeitsmatrix $\Pi$ dargestellt, wobei jedes Element $\pi_{qi}$ in der Matrix Anfangswahrscheinlichkeit des verborgenen Zustands $q_i$ darstellt, d. h. die Werkseinstellungswahrscheinlichkeitsverteilung;

S220, Einrichten eines Hidden-Markow-Modells, das $\lambda = \{A, B, \Pi\}$ ist;

S230, Durchführen einer Hidden-Markow-Prozessdarstellung, wobei die Hidden-Markow-Prozessdarstellung die Ausgabewahrscheinlichkeit der Feuchtigkeits- und Gasdruckbeobachtungssequenz $O$ nach dem Modell $\lambda = \{A, B, \Pi\}$ ist, wobei $P(O|\lambda)$ eine Verbundwahrscheinlichkeit darstellt, dass die Feuchtigkeits- und Gasdruckbeobachtungssequenz O und die verborgene Gasdichtheitszustandssequenz $Q = [q_1, q_2,..., q_T]$ nach dem Modell $\lambda$ gleichzeitig auftreten, wobei die Formel wie folgt lautet:

$$P(O|\lambda) = \sum_O P(O|Q,\lambda) = \sum_{q_1,q_2...q_T} \pi_{q_1} b_{q_1}(o_1) a_{q_1 q_2} b_{q_2}(o_2)...a_{q_{T-1} q_T} b_{q_T}(o_T)$$

,

wobei für einen festen verborgenen Zustand $Q = [q_1, q_2,..., q_T]$ die Wahrscheinlichkeit, eine Feuchtigkeits- und Gasdruckbeobachtungssequenz $O = [o_1, o_2,..., o_T]$ zu erhalten, $P(O|Q, \lambda) = b_{q1}(o_1)b_{q2}(o_2)...b_{qT}(o_T)$ ist.

6. Verfahren zum Schätzen von Defibrillationselektrodenverschleiß nach Anspruch 5, wobei der Schritt S300 des Lernens für das Hidden-Markov-Modell Folgendes umfasst:

S310, Sammeln von K Gruppen von Feuchtigkeits- und Gasdruckbeobachtungssequenzen $O = [O^1, O^2,..., O^K]$,

wobei $O^k = [O_1^k, O_2^k, ..., O_T^k]$ die k-te Gruppe Feuchtigkeits- und Gasdruckbeobachtungssequenz darstellt;

S320, Erhalten der Modellparameter, die den maximalen Verbundwahrscheinlichkeitswert unter der k-ten Gruppe Feuchtigkeits- und Gasdruckbeobachtungssequenz $O^k$ ausmacht, und Definieren der Modellparameter als optimal;

S330, Trainieren unter Verwendung von K Gruppen von Feuchtigkeits- und Gasdruckbeobachtungssequenzen, um den optimalen Parametersatz mit der gegebenen Feuchtigkeits- und Gasdruckbeobachtungssequenz O zu erhalten;

S340, durch Anpassen Erreichen, dass die Verbundwahrscheinlichkeit, dass die Feuchtigkeits- und Gasdruckbeobachtungssequenz **O** und die verborgene Gasdichtheitszustandssequenz $Q = [q_1, q_2,..., q_T]$ gleichzeitig nach dem Modell $\lambda$ auftreten, maximal ist, und Einstellen der optimalen Modellparameter, die mit jeder Feuchtigkeits- und Gasdruckbeobachtungssequenz erhalten werden, als bestimmte Modellparameter.

7. Verfahren zum Schätzen von Defibrillationselektrodenverschleiß nach Anspruch 6, wobei in Schritt S400 der Schritt zum Erhalten verborgener Parameter in Bezug auf Gasdichtheit, die der verborgenen Gasdichtheitszustandssequenz entspricht, Folgendes umfasst:

S410, unter den bestimmten Modellparametern, Definieren von $\delta_t(i)$ als maximale Wahrscheinlichkeit zum Erzeugen von $o_1$, $o_2$,..., $o_t$ entlang eines Pfades $q_1$, $q_2$,..., $q_T$ zum Zeitpunkt t;

S420, Initialisieren von $\delta_t(i) = \pi_i b_i(o_i)$:

S430, Berechnen der Ergebnisse $\delta_t(j) = \max_i (\delta_{t-1}(i) a_{ij} | b_j(o_t))$ der Feuchtigkeits- und Gasdruckbeobachtungssequenz, die der vorherigen Feuchtigkeits- und Gasdrucksequenz auf dem nächsten Pfad j entspricht;

S440, Heranziehen des Maximalwerts in dem Pfad in dem Schritt S430 als das Ausgabeergebnis

$$P(Q|\lambda) = \max_i (\delta_{t-1}(i) a_{ij})$$, wobei $P(Q|\lambda)$ der Parameter ist, der durch die Klimavariable C und die Aktivierungsenergieausfallmechanismusvariable E unter dem Einfluss von Gasdichtheit dargestellt ist.

8. Defibrillationselektrodenanordnung zur intelligenten Anzeige von Defibrillationselektrodenverschleiß, wobei die Anordnung ein Klimabehältnis, eine Defibrillationselektrode, eine Inselelektrode und ein Detektionsmodul umfasst, wobei die Defibrillationselektrode und die Inselelektrode innerhalb des Klimabehältnisses angeordnet sind und das Detektionsmodul einen Feuchtigkeitssensor, einen Gasdrucksensor und einen Temperatursensor umfasst, die in dem Klimabehältnis angeordnet sind, wobei das Detektionsmodul dazu konfiguriert ist, den Defibrillationselektrodenverschleiß durch Vollführen des Verfahrens zum Schätzen von Defibrillationselektrodenverschleiß nach einem der Ansprüche 1 bis 7 unter Verwendung von Parametern, die von dem Feuchtigkeitssensor, dem Drucksensor und dem Temperatursensor erhalten werden, zu schätzen, und dazu konfiguriert ist, das Schätzergebnis über ein Anzeigemodul anzuzeigen.

9. Defibrillationselektrodenanordnung nach Anspruch 8, wobei das Detektionsmodul eine Steuerung umfasst, der Feuchtigkeitssensor über ein Datenverarbeitungsmodul mit der Steuerung verbunden ist, der Temperatursensor in Reihe mit einem Widerstandsspannungsteilermodul verbunden ist und mit der Steuerung verbunden ist und der Gasdrucksensor mit der Steuerung verbunden ist, vorzugsweise die Inselelektrode zwei Testbögen aus dem gleichen Material wie dasjenige der Defibrillationselektrode umfasst, die Defibrillationsschichten der zwei Testbögen entgegengesetzt gestapelt sind und die Impedanz-Testpunkte der zwei Testbögen mit dem Detektionsmodul verbunden sind, um eine Impedanz-Detektionsschaltung für eingegebenen Gleichstrom auszubilden.

10. Lesbares Speichermedium, wobei ein Steuerprogramm auf dem lesbaren Speichermedium gespeichert ist und das Steuerprogramm, wenn es durch das Detektionsmodul der Defibrillationselektrodenanordnung nach Anspruch 8 oder Anspruch 9 ausgeführt wird, das Detektionsmodul befähigt, die Schritte des Verfahrens nach einem der Ansprüche 1 bis 7 auszuführen.

11. Defibrillationselektrodenanordnung nach Anspruch 8 oder 9, die zwei Defibrillationselektroden umfasst, die sich in einem versiegelten Verpackungsbeutel befinden,

wobei die zwei Defibrillationselektroden über einen Defibrillationsentladungs-Hochspannungsdraht mit einem externen Anschlussende verbunden sind;

wobei die Inselelektrode zur Rückmeldung über die Leistungsparameter der Defibrillationselektrode verwendet wird, die Inselelektrode in dem versiegelten Verpackungsbeutel angeordnet ist und die Inselelektrode mit dem Detektionsmodul ausgestattet ist, das mit dem externen Anschlussende verbunden ist.

**12.** Defibrillationselektrodenanordnung nach Anspruch 11, wobei die Inselelektrode zwei Testbögen aus dem gleichen Material wie dasjenige der Defibrillationselektrode umfasst und die Defibrillationsschichten der zwei Testbögen entgegengesetzt überlappt sind;

wobei das Detektionsmodul einen Impedanz-Testpunkt umfasst, der sich auf der Defibrillationsschicht befindet, und die Impedanz-Testpunkte der zwei Testbögen mit dem externen Anschlussende verbunden sind, um eine Impedanz-Detektionsschaltung für eingegebenen Wechselstrom auszubilden.

**13.** Defibrillationselektrodenanordnung nach Anspruch 12, wobei das Detektionsmodul über einen Detektionsdraht mit dem externen Anschlussende verbunden ist, sich das externe Anschlussende an der Außenseite des versiegelten Verpackungsbeutels befindet und sich der Detektionsdraht von dem versiegelten Verpackungsbeutel erstreckt und mit dem externen Anschlussende verbunden ist.

**14.** Defibrillationselektrodenanordnung nach Anspruch 13, wobei die Fläche der Inselelektrode kleiner als diejenige der Defibrillationselektrode ist,

sich vorzugsweise das externe Anschlussende auf einer Seite des versiegelten Verpackungsbeutels befindet und das Inselelektrodenstück nahe dem externen Anschlussende positioniert ist.

**15.** Defibrillationsvorrichtung, wobei die Defibrillationsvorrichtung eine Defibrillationselektrodenanordnung nach einem der Ansprüche 8 bis 14 und einen Defibrillator, der elektrisch mit der Defibrillationselektrodenanordnung verbunden ist, aufweist.

**Revendications**

**1.** Procédé permettant l'estimation de l'usure d'une électrode de défibrillation, comprenant :

S100, mise en place d'un conteneur environnemental pour estimer l'atténuation électrochimique d'un matériau de gel de l'électrode de défibrillation, et détermination des variables environnementales et des variables du mécanisme de défaillance d'énergie d'activation comme paramètres cachés liés à l'étanchéité au gaz, pour estimer l'atténuation électrochimique du matériau de gel de l'électrode de défibrillation, afin d'estimer l'usure de l'électrode de défibrillation, dans lequel le conteneur environnemental est équipé d'un capteur d'humidité et d'un capteur de pression de gaz, pour observer les séquences d'humidité et de pression de gaz ;

S200, établissement d'un modèle de Markov caché et utilisation de celui-ci pour établir un processus de transition d'état pour les paramètres cachés liés à l'étanchéité au gaz, sous les séquences d'humidité et de pression de gaz observées, dans lequel les valeurs observées des séquences d'humidité et de pression de gaz sont utilisées comme vecteurs d'observation de la chaîne de Markov cachée et sont définies comme un ensemble de séquences d'observation d'humidité et de pression de gaz, et les paramètres cachés liés à l'étanchéité au gaz sont utilisés comme vecteurs d'état caché de la chaîne de Markov cachée et sont définis comme des séquences d'état caché d'étanchéité au gaz ;

S300, apprentissage pour le modèle de Markov caché et ajustement des paramètres du modèle à l'aide de multiples ensembles collectés de séquences d'observation d'humidité et de pression de gaz, dans lequel un ensemble de paramètres de modèle optimaux obtenus sous chaque séquence d'observation d'humidité et de pression de gaz est déterminé en tant que paramètres de modèle déterminés ;

S400, décodage du modèle de Markov caché et estimation de l'usure de l'électrode de défibrillation, dans lequel lors de l'utilisation d'un dispositif de défibrillation, le décodage est basé sur les paramètres de modèle déterminés et les séquences d'observation d'humidité et de pression de gaz correspondant aux séquences d'humidité et de pression de gaz à un moment précédent, de manière à obtenir des paramètres cachés liés à l'étanchéité au gaz correspondant à la séquence d'état caché d'étanchéité au gaz actuelle, dans lequel les variables environnementales et les variables de mécanisme de défaillance d'énergie d'activation correspondant aux paramètres cachés liés à l'étanchéité au gaz sont utilisées pour estimer la mesure de durée de vie du matériau de gel de l'électrode de défibrillation, et une valeur actuelle estimée de l'usure de l'électrode de défibrillation est finalement obtenue et affichée.

**2.** Procédé permettant l'estimation de l'usure d'une électrode de défibrillation selon la revendication 1, dans lequel à l'étape S100, le conteneur environnemental est en outre équipé d'un capteur de température pour observer des valeurs de température et dans lequel à l'étape S400, la valeur de l'usure de l'électrode de défibrillation est estimée en outre sur la base des valeurs de température.

**3.** Procédé permettant l'estimation de l'usure d'une électrode de défibrillation selon la revendication 2, dans lequel la formule de réaction suivante est utilisée pour estimer la perte de durée de vie de l'électrode principalement sur la base de la perte d'énergie d'activation du matériau de gel de l'électrode de défibrillation :

$$F = Ce^{-\frac{E}{kB*M}}$$

où F est la vitesse de réaction chimique du matériau de gel de l'électrode de défibrillation, et la durée de vie de l'électrode de défibrillation peut être calculée comme l'inverse de la vitesse de réaction chimique du matériau de gel de l'électrode de défibrillation, c'est-à-dire 1/F ; C représente la variable environnementale : e représente la base logarithmique naturelle ; E représente la variable du mécanisme de défaillance d'énergie d'activation ; kB est la constante de Boltzmann ; M représente la température.

**4.** Procédé permettant l'estimation de l'usure d'une électrode de défibrillation selon la revendication 3, dans lequel l'étape S200 comprend :

S210, définition de chaque tuple dans le modèle de Markov caché, dans lequel chaque tuple comprend un ensemble de séquences d'observation d'humidité et de pression de gaz, un ensemble d'états cachés, une matrice de probabilité de transition d'état, une matrice de probabilité d'émission et une matrice de probabilité initiale ;
S220, établissement d'un modèle de Markov caché, qui est lié à la matrice de probabilité de transition d'état, à la matrice de probabilité d'émission et à la matrice de probabilité initiale ;
S230, réalisation d'une représentation de processus de Markov caché, ladite représentation de processus de Markov caché étant une probabilité de sortie des séquences d'observation d'humidité et de pression de gaz sous le modèle de Markov caché, c'est-à-dire une probabilité conjointe que la séquence d'observation d'humidité et de pression de gaz et la représentation d'état caché d'étanchéité au gaz se produisent simultanément sous le modèle de Markov caché.

**5.** Procédé permettant l'estimation de l'usure d'une électrode de défibrillation selon la revendication 4, dans lequel l'étape S200 comprend :

S210, définition des tuples dans le modèle de Markov caché ;
S211, définition d'un ensemble de séquences d'observation d'humidité et de pression de gaz, à l'aide des séquences d'observation d'humidité et de pression de gaz $O = [o_1, o_2,...,o_T]$ de la séquence temporelle d'humidité et la séquence temporelle de pression de gaz mesurées en temps réel comme vecteur d'observation de la chaîne de Markov cachée, et définition d'un ensemble de séquences d'observation d'humidité et de pression de gaz, où T représente une période pendant laquelle le capteur d'humidité et le capteur de pression de gaz commencent à mesurer à un temps fixe lorsque le dispositif de défibrillation fonctionne, $o_t = [pression_t, humidité_t]^T$ est la valeur d'observation de la pression de gaz et de l'humidité mesurée à l'instant t ;
S212, définition d'un ensemble d'états cachés, en considérant la représentation d'état caché d'étanchéité au gaz comme une séquence d'état caché d'étanchéité au gaz Q de la chaîne de Markov, et définition de la séquence d'état caché d'étanchéité au gaz comme $Q = [q_1, q_2,..., q_T]$, qt = $[Ct, E_t]^T$ étant la représentation cachée des paramètres cachés liés à l'étanchéité au gaz à l'instant t ;
S213, définition d'une matrice de probabilité de transition d'état, dans laquelle pour l'ensemble de transition d'état des états cachés d'étanchéité au gaz à différents instants, elle est représentée comme une matrice de probabilité de transition d'état A, dans laquelle chaque élément $a_{ij}$ de la matrice de probabilité de transition d'état A représente la probabilité de transition de l'état i à l'état j ;
S214, définition d'une matrice de probabilité d'émission : pour l'ensemble des valeurs de mesure d'humidité et de pression de gaz possibles générées dans l'état caché d'étanchéité au gaz, elle est représentée comme une matrice de probabilité d'émission B, dans laquelle l'élément $b_{qi}$ de la matrice de probabilité d'émission B représente la probabilité d'émission depuis un état caché $q_i$ à un état d'observation ;
S215, définition d'une matrice de probabilité initiale : pour l'expression du défaut d'usine de l'état caché d'étanchéité au gaz initial, elle est représentée comme une matrice de probabilité initiale $\Pi$, dans laquelle

chaque élément $\pi_{qi}$ dans la matrice représente la probabilité initiale de l'état caché $q_i$, c'est-à-dire la distribution de probabilité de défaut d'usine ;

S220, établissement d'un modèle de Markov caché, qui est $\lambda = \{A, B, \Pi\}$ ;

S230, réalisation d'une représentation de processus de Markov caché, dans laquelle la représentation de processus de Markov caché est la probabilité de sortie de la séquence d'observation d'humidité et de pression de gaz **O** sous le modèle $\lambda = \{A, B, \Pi\}$, dans laquelle $P(O|\lambda)$ représente une probabilité conjointe que la séquence d'observation d'humidité et de pression de gaz **O** et la séquence d'état caché d'étanchéité au gaz **Q** = [$q_1$, $q_2$,..., $q_T$] se produisent simultanément sous le modèle $\lambda$, dans laquelle la formule est la suivante :

$$P(O|\lambda) = \sum_O P(O|Q,\lambda) = \sum_{q_1,q_2\ldots q_T} \pi_{q_1} b_{q_1}(o_1) a_{q_1 q_2} b_{q_2}(o_2)\ldots a_{q_{T-1}q_T} b_{q_T}(O_T)$$

pour un état caché fixe **Q** = [$q_1$, $q_2$,..., $q_T$], la probabilité d'obtenir une séquence d'observation d'humidité et de pression de gaz **O** = [$o_1$, $o_2$,..., $o_T$] est $P(O|Q, \lambda) = b_{q1}(o_1)b_{q2}(o_2)\ldots b_{qT}(o_T)$.

6. Procédé permettant l'estimation de l'usure d'une électrode de défibrillation selon la revendication 5, dans lequel l'étape S300 d'apprentissage du modèle de Markov caché comprend :

S310, collecte de K groupes de séquences d'observation d'humidité et de pression de gaz **O** = [**O¹, O²,..., Oᴷ**],

dans laquelle $O^k = [O_1^k, O_2^k, \ldots, O_T^k]$ représente la kth séquence d'observation d'humidité et de pression de gaz du groupe ;

S320, obtention des paramètres du modèle qui maximisent la valeur de probabilité conjointe sous la kth séquence d'observation d'humidité et de pression de gaz de groupe $O^k$, et définition des paramètres du modèle comme optimaux ;

S330, apprentissage à l'aide des K groupes des séquences d'observation d'humidité et de pression de gaz pour obtenir l'ensemble de paramètres optimal avec la séquence d'observation d'humidité et de pression de gaz donnée O ;

S340, par ajustement, maximisation de la probabilité conjointe que la séquence d'observation d'humidité et de pression de gaz **O** et la séquence d'état caché d'étanchéité aux gaz **Q** = [$q_1$, $q_2$,..., $q_T$] se produisent simultanément sous le modèle $\lambda$, et définition des paramètres de modèle optimaux obtenus avec chaque séquence d'observation d'humidité et de pression de gaz en tant que paramètres de modèle déterminés.

7. Procédé permettant l'estimation de l'usure d'une électrode de défibrillation selon la revendication 6, dans lequel à l'étape S400, l'étape d'obtention de paramètres cachés liés à l'étanchéité au gaz correspondant à la séquence d'états cachés d'étanchéité au gaz comprend :

S410, parmi les paramètres du modèle déterminés, définition de $\delta_t(i)$ comme probabilité maximale pour générer $o_1$, $o_2$,..., $o_t$ le long d'un trajet $q_1$, $q_2$,..., $q_T$ à l'instant t ;

S420, initialisation de $\delta_t(i) = \pi_i b_i(oi)$ ;

S430, calcul des résultats $\delta_t(j) = \max_i (\delta_{t-1}(i)a_{ij}|b_j(o_t))$ de la séquence d'observation d'humidité et de pression de gaz correspondant à la séquence d'humidité et de pression de gaz précédente sur le trajet suivant j ;

S440, considération de la valeur maximale sur le trajet à l'étape S430 comme résultat de sortie $P(Q|\lambda) = \max_i (\delta_{t-1}(i)a_{ij})$, dans laquelle $P(Q|\lambda)$ est le paramètre représenté par la variable environnementale C et la variable de mécanisme de défaillance d'énergie d'activation E sous l'influence de l'étanchéité au gaz.

8. Ensemble électrode de défibrillation pour un affichage intelligent de l'usure d'une électrode de défibrillation, dans lequel l'ensemble comprend un conteneur environnemental, une électrode de défibrillation, une électrode îlot et un module de détection, dans lequel l'électrode de défibrillation et l'électrode îlot sont placées à l'intérieur du conteneur environnemental, et le module de détection comprend un capteur d'humidité, un capteur de pression de gaz et un capteur de température placés dans le conteneur environnemental, dans lequel le module de détection est configuré pour estimer l'usure de l'électrode de défibrillation en mettant en œuvre le procédé d'estimation de l'usure de l'électrode de défibrillation selon l'une quelconque des revendications 1 à 7 à l'aide des paramètres obtenus à partir du capteur d'humidité, du capteur de pression et du capteur de température, et est configuré pour afficher le résultat d'estimation via un module d'affichage.

9. Ensemble électrode de défibrillation selon la revendication 8, dans lequel le module de détection comprend un contrôleur, le capteur d'humidité est connecté au contrôleur par l'intermédiaire d'un module de traitement de données, le capteur de température est connecté en série avec un module diviseur de tension à résistance et connecté au contrôleur, et le capteur de pression de gaz est connecté au contrôleur,
de préférence, l'électrode îlot comprend deux feuilles de test du même matériau que celui de l'électrode de défibrillation, les couches de défibrillation des deux feuilles de test sont empilées de manière opposée, et les points de test d'impédance des deux feuilles de test sont connectés au module de détection pour former un circuit de détection d'impédance pour le courant continu d'entrée.

10. Support de stockage lisible, dans lequel un programme de commande est stocké sur le support de stockage lisible, et le programme de commande permet, lorsqu'il est exécuté par le module de détection de l'ensemble électrode de défibrillation selon la revendication 8 ou la revendication 9, au module de détection d'exécuter les étapes du procédé selon l'une quelconque des revendications 1 à 7.

11. Ensemble électrode de défibrillation selon la revendication 8 ou 9, qui comprend deux électrodes de défibrillation situées dans un sachet de conditionnement scellé,

dans lequel les deux électrodes de défibrillation sont connectées à une extrémité de connexion externe par l'intermédiaire d'un fil haute tension de décharge de défibrillation ;
dans lequel l'électrode îlot est utilisée pour une rétroaction sur les paramètres de performance de l'électrode de défibrillation, l'électrode îlot est placée dans le sachet de conditionnement scellé, et l'électrode îlot est équipée du module de détection, qui est connecté à l'extrémité de connexion externe.

12. Ensemble électrode de défibrillation selon la revendication 11, dans lequel l'électrode îlot comprend deux feuilles de test du même matériau que celui de l'électrode de défibrillation, et les couches de défibrillation des deux feuilles de test se chevauchent de manière opposée ;
dans lequel le module de détection comprend un point de test d'impédance situé sur la couche de défibrillation, et les points de test d'impédance des deux feuilles de test sont connectés à l'extrémité de connexion externe pour former un circuit de détection d'impédance pour le courant alternatif d'entrée.

13. Ensemble électrode de défibrillation selon la revendication 12, dans lequel le module de détection est connecté à l'extrémité de connexion externe par l'intermédiaire d'un fil de détection, l'extrémité de connexion externe est située à l'extérieur du sachet de conditionnement scellé, et le fil de détection s'étend à partir du sachet de conditionnement scellé et est connecté à l'extrémité de connexion externe.

14. Ensemble électrode de défibrillation selon la revendication 13, dans lequel la surface de l'électrode îlot est inférieure à celle de l'électrode de défibrillation,
de préférence, l'extrémité de connexion externe est située sur un côté du sachet de conditionnement scellé, et la pièce d'électrode îlot est agencée à proximité de l'extrémité de connexion externe.

15. Dispositif de défibrillation, dans lequel le dispositif de défibrillation comporte un ensemble électrode de défibrillation selon l'une quelconque des revendications 8 à 14, et un défibrillateur connecté électriquement à l'ensemble électrode de défibrillation.

Setting an environmental container for estimating electrochemical attenuation of gel materials — S100

Establishing a hidden Markov model — S200

Learning for the hidden Markov model — S300

Decoding the hidden Markov model and estimating the loss of defibrillation electrode — S400

Fig.1

$O = [o_1, o_2 ..., o_T]$: Timely measured gas pressure and humidity

$Q = [q_1, q_2 ..., q_T]$: Gas tightness hidden state representation

Fig.2

Fig.3

Fig.4

Fig.5

Fig.6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311263066
- CN 202311826856X
- US 2005277991 A1